# EUROPÄISCHE PATENTSCHRIFT

(11) **EP 3 038 752 B1**
(45) Veröffentlichungstag und Bekanntmachung des Hinweises auf die Patenterteilung: **19.07.2017**
(21) Anmeldenummer: 14750354.4
(22) Anmeldetag: 08.08.2014
(51) Int. Cl.: B01J 31/18, B01J 31/02, B01J 21/02, B01J 21/06, B01J 21/08, B01J 21/18, C07C 45/50, B01J 31/40

(54) **KATALYSATOR UND VERFAHREN ZUR HYDROFORMYLIERUNG VON UNGESÄTTIGTEN VERBINDUNGEN DURCH SILP-KATALYSE**
CATALYST AND METHOD FOR THE HYDROFORMYLATION OF UNSATURATED COMPOUNDS BY MEANS OF SILP CATALYSIS
CATALYSEUR ET PROCÉDÉ D'HYDROFORMYLATION DE COMPOSÉS INSATURÉS PAR CATALYSE SILP

(30) Priorität: 28.08.2013 DE 102013217166
(43) Veröffentlichungstag der Anmeldung: 06.07.2016
(73) Patentinhaber: Evonik Degussa GmbH, 45128 Essen (DE)
(72) Erfinder: DYBALLA, Katrin, Marie, 45657 Recklinghausen (DE); FRANKE, Robert, 45772 Marl (DE); HAHN, Hanna, 47199 Duisburg-Baerl (DE)
(86) Internationale Anmeldenummer: PCT/EP2014/067072
(87) Internationale Veröffentlichungsnummer: WO 2015/028284

(56) Entgegenhaltungen:
- EP-A1- 0 472 071
- EP-A1- 1 201 675
- EP-A1- 2 003 138
- EP-A2- 0 214 622
- WO-A1-02/00670
- WO-A1-2012/041846
- WO-A1-2014/056736
- DE-A1- 19 602 301
- DE-A1- 19 631 521
- DE-A1-102011 085 883
- US-A- 5 672 766
- US-A- 5 767 321
- US-A- 5 962 744
- PACIELLO ROCCO ET AL: "Structure-activity relationship for chelating phosphite ligands used in rhodium-catalyzed hydroformylations", JOURNAL OF MOLECULAR CATALYSIS A: CHEMICAL, ELSEVIER, AMSTERDAM, NL, Bd. 143, Nr. 1-3, 1. Januar 1999 (1999-01-01), Seiten 85-97, XP002499694, ISSN: 1381-1169, DOI: 10.1016/S1381-1169(98)00370-7

## Beschreibung

Gegenstand der vorliegenden Erfindung ist eine Zusammensetzung, umfassend: a) mindestens ein Trägermaterial; b) mindestens eine Ionische Flüssigkeit; c) mindestens ein Metall, ausgewählt aus der 9. Gruppe des Periodensystems der Elemente; d) mindestens eine Verbindung der Formel (I) R'-A-R" (I), wobei A, R' und R" jeweils ein organischer Rest, wobei R' und R" das Strukturelement -O-P(-O-)₂ mit dreiwertigem P aufweisen und über dieses mit dem Rest A kovalent verknüpft sind, mit der Maßgabe, dass R' ≠ R" und e) optional ein oder mehrere organische Amine, ein Verfahren zur Herstellung einer solchen Zusammensetzung, die Verwendung der Zusammensetzung sowie ein Verfahren zur Hydroformylierung, bei der die Zusammensetzung verwendet wird.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxierung bekannt (Schema 1). Als Katalysatoren in diesen Reaktionen werden häufig Verbindungen der Übergangsmetalle der VIII. Gruppe des Periodensystems der Elemente verwendet, insbesondere Rhodium- oder Cobaltkatalysatoren. Bekannte Liganden sind beispielsweise Verbindungen aus den Klassen der Phosphine, Phosphite und Phosphonite mit jeweils dreiwertigem Phosphor P^{III}. Eine gute Übersicht über den Stand der Hydroformylierung von Olefinen findet sich in B. CORNILS, W. A. HERRMANN, "Applied Homogeneous Catalysis with Organometallic Compounds", Vol. 1 & 2, VCH, Weinheim, New York, 1996 bzw. R. Franke, D. Selent, A. Börner, "Applied Hydroformylation", Chem. Rev., 2012, DOI:10.1021/cr3001803.

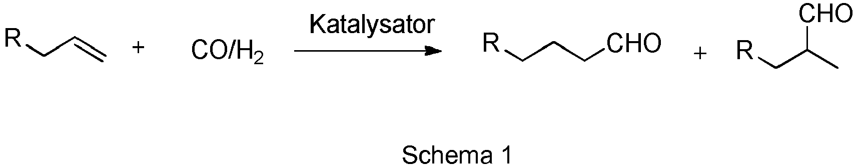

Bei der Entwicklung neuer Hydroformylierungsverfahren wurde von der Idee ausgegangen, die bislang homogen in der Reaktionsmischung vorliegenden, Rhodium aufweisenden Katalysatorsysteme zu immobilisieren. Es kann in diesem Zusammenhang von der Heterogenisierung einer an sich homogen durchgeführten Reaktion - in diesem Fall der Hydroformylierung - gesprochen werden.

In den letzten Jahrzehnten sind zahlreiche Techniken zur Immobilisierung homogener Katalysatoren entwickelt und viele dieser Konzepte für Hydroformylierungsreaktion angewendet worden (siehe: M. Beller, B. Cornils, C. D. Frohning, C. W. Kohlpaintner, J. Mol. Catal. 1995, 104, 17).

Die Heterogenisierung der Katalysatorkomplexe durch Immobilisierung auf porösen Trägermaterialien ist eingehend untersucht worden. Solche Heterogenisierung kann z. B. durch kovalente Verankerung des Rhodium-Komplexes über Spacer-Liganden auf dem Träger erreicht werden (siehe: V. A. Likholobov, B. L. Moroz, Hydroformylation on Solid Catalysts in: Handbook of Heterogeneous Catalysis, 2nd ed.; G. Ertl, H. Knoezinger, F. Schüth, J. Weitkamp (Eds.), Wiley-VCH, Weinheim, Germany, 2008, 3663).

Neben dem Supported-Aqueous-Phase (SAP) Konzept (siehe H. Delmas, U. Jaeuregui-Haza, A.-M. Wilhelm, Supported Aqueous-Phase Catalysis as the Alternative Method in: Multiphase Homogeneous Catalysis, B. Cornils, W. A. Herrmann, I. T. Horväth, W. Leitner, S. Mecking, H. Olivier-Bourbigou, D. Vogt (Eds.), Wiley-VCH, Weinheim, Germany, 2005), welches aber für hydrolyseempfindliche Liganden ungeeignet ist, stellt das sogenannte Supported-Liquid-Phase (SLP) Konzept ein weiteres Konzept zur Heterogenisierung von homogenen Katalysatorkomplexen dar: Hierbei wird eine flüssige Katalysatorlösung auf ein poröses Trägermaterial aufgebracht. Dieses Konzept ist bereits über 40 Jahre bekannt (siehe: P. Rony, J. Catal. 1969, 14, 142; G.J.K. Acres, G.C. Bond, B.J. Cooper, J.A. Dawson, J. Catal. 1969, 6, 139). Für die Hydroformylierung werden unter anderem geschmolzene Salze wie z.B. Triphenylphosphan (TPP) als flüssige Phase eingesetzt. TPP dient hierbei als Lösungsmittel für den Katalysatorkomplex, aber auch als Ligand und wird daher in einem großen Überschuss eingesetzt. Problematisch an einem sehr großen Ligandenüberschuss bei betrachteten Katalysatorsystemen ist die Bildungen verschiedener Übergangsmetallkomplexe, die eine Unterdrückung der katalytischen Aktivität zur Folge haben können.

Nach Literaturangaben leiden jedoch rein heterogene Katalysatoren unter einer niedrigen Hydroformylierungsaktivität, zeichnen sich jedoch durch eine in diesem Fall unerwünschte recht hohe Hydrieraktivität aus (siehe: a) M. E. Davis, E. Rode, D. Taylor, B. E. Hanson, J. Catal.1984, 86, 67; b) S. Naito, M. Tanimoto, J. Chem. Soc. Chem. Commun. 1989, 1403; c) G. Srinivas, S. S. C. Chung, J. Catal. 1993, 144, 131). Ohne das Vorhandensein einer flüssigen Reaktionsphase, in der der metallorganische Katalysatorkomplex gelöst vorliegt, wird oft eine schlechte Regioselektivität festgestellt.

Die bislang aussichtsreichste Entwicklung ist die Hydroformylierung von Olefinen zu Aldehyden mittels sogenannter Supported-Ionic-Liquid-Phase-, kurz genannt SILP-Katalysatorsystemen. Dies sind katalytisch wirksame Zusammensetzungen in einem Mehrphasensystem, die aus einem festen, inerten, porösen Trägermaterial bestehen, das mit einer Ionischen Flüssigkeit, kurz IL genannt, umhüllt ist - der sogenannten SILP-Phase - in welcher der Übergangsmetall-, insbesondere Rhodium, aufweisende Katalysator enthalten ist (siehe: a) A. Riisager, P. Wasserscheid, R. van Hal, R. Fehrmann, J. Catal. 2003, 219, 252; b) M. Haumann, K. Dentler, J. Joni, A. Riisager, P. Wasserscheid, Adv. Synth. Catal. 2007, 349, 425; c) S. Shylesh, D. Hanna, S. Werner, A. T. Bell, ACS Catal. 2012, 2, 487; d) M. Jakuttis, A. Schoenweiz, S. Werner, R. Franke, K.-D. Wiese, M. Haumann, P. Wasserscheid, Angew. Chem. Int. Ed. 2011, 50, 4492).

Mit SILP-Katalysatorsystemen lassen sich die Vorteile von homogen und heterogen katalysierten Synthesereaktionen vereinen. Dies betrifft vor allem die Produktabtrennung und Rückgewinnung des Katalysators, insbesondere der darin enthaltenen Übergangsmetalle, welche sich bei homogen geführten Synthesereaktionen als schwierig und aufwendig darstellt. Bei heterogen katalysierten Synthesereaktionen kann es hingegen zu Massen- und Wärmetransportlimitierung kommen, wodurch sich die Aktivität des festen Katalysatorsystems verringert; auch werden bei heterogen katalysierten Synthesereaktionen geringere Chemo- und Stereoselektivitäten beobachtet.

Für den wirtschaftlichen Betrieb eines kontinuierlichen Verfahrens zur Hydroformylierung ist nicht allein die Nutzung eines sehr aktiven und selektiven Katalysatorsystems von Bedeutung. Besonders die Punkte Katalysatorrecycling - verbunden mit der Produktabtrennung - und Ligandenstabilität spielen eine entscheidende Rolle - nicht nur angesichts der hohen Rhodium- und Ligandenpreise, sondern auch des nur ansatzweise bekannten Einflusses von Verunreinigungen aus Liganden-Abbauprozessen auf die Aktivität und das Produktspektrum.

Allgemein bekannt ist, dass Liganden auf Basis von Organophosphiten in der Hydroformylierung einem inhärenten Abbau- und Desaktivierungsprozess unterliegen. [P. W. N. M. van Leeuwen, in Rhodium Catalyzed Hydroformylation, P. W. N. M. van Leeuwen, C. Claver (Hrsg.), Kluwer, Dordrecht, 2000.]

Da einige Katalysatorgifte wie Wasser, Alkohole, Ameisensäure, Sauerstoff oder Peroxide in Spuren immer in einem Hydroformylierungsprozess gebildet werden bzw. technisch nicht vermeidbar sind, z.B. durch Folgereaktionen der Aldehyde, wie beispielsweise der Aldolkondensation, Folgereaktionen des Synthesegases, spricht man von einer inhärenten, systembedingten Instabilität des Katalysatorssystems und dessen Einzelkomponenten.

Neben- und Abbaureaktionen können beispielsweise Hydrolyse, Alkoholyse, Umesterung, Arbusov-Umlagerung, P-O-Bindungsspaltung und P-C-Bindungsspaltung sein [P. W. N. M. van Leeuwen, in Rhodium Catalyzed Hydroformylation, P. W. N. M. van Leeuwen, C. Claver (Hrsg.), Kluwer, Dordrecht, 2000.; F. Ramirez, S. B. Bhatia, C. P. Smith, Tetrahedron 1967, 23, 2067-2080.; E. Billig, A. G. Abatjoglou, D. R. Bryant, R. E. Murray, J. M. Maher, (Union Carbide Corporation), US Pat. 4,789,753 1988; M. Takai, I. Nakajima, T. Tsukahara, Y. Tanaka, H. Urata, A. Nakanishi, EP 1 008 581 B1 2004.].

Literaturbekannte Ligandensysteme für die Gasphasenhydroformylierung mit SILP-Systemen sind u.a. Bisphosphite (DE 102010041821), Mono- und Bisphosphine (Riisager et al., Catal. Lett. 2003, 90, 149.; Riisager et al. J. Catal. 2003, 219, 452). Bisphosphite zeichnen sich durch eine hohe Langzeitstabilität von 700 h bei einer Reaktionstemperatur von 100°C aus.

In vielen technischen Strömen sind neben terminalen Alkenen mit endständigen Doppelbindungen häufig auch interne Olefine, die innenständige Doppelbindungen aufweisen, zu finden. Daher ist es wichtig eine katalytisch aktive Zusammensetzung zu entwickeln, die neben der Umsetzung von terminalen Alkenen auch zur Umsetzung in einer isomerisierenden Hydroformylierung geeignet ist. Beispielsweise die literaturbekannten Liganden des Benzpinacol-Typs sind jedoch nicht in einer isomerisierenden Hydroformylierung aktiv, wie DE 102006058682 offenbart.

Aufgabe der vorliegenden Erfindung war die Bereitstellung eines Katalysatorsystems für die Hydroformylierung welches einen oder mehrere der Nachteile der Katalysatorsysteme des Standes der Technik nicht aufweist.

Insbesondere war Aufgabe der vorliegenden Erfindung ein Verfahren bereitzustellen, welches sowohl eine günstige Katalysatorabtrennung ermöglicht, als auch unter Isomerisierung ungesättigte Verbindungen zu n-terminalen Aldehyden zu hydroformylieren vermag und vorzugsweise gleichzeitig eine verbesserte Katalysatorstandzeit im Vergleich zu den im Stand der Technik beschriebenen Systemen aufweist.

Überraschenderweise wurde gefunden, dass diese Aufgabe gelöst werden kann durch eine Zusammensetzung gemäß Anspruch 1, die einen Katalysatorkomplex gelöst in einer Ionischen Flüssigkeit auf einem heterogenen Träger aufweist, der sogenannten SILP-Phase, wobei der Komplex einen unsymmetrisch substituierten Bisphosphit-Liganden aufweist.

Insbesondere überraschend ist in diesem Zusammenhang, dass unsymmetrisch aufgebaute Bisphosphite die Aufgabe lösen. Aus dem Stand der Technik ist bekannt, dass unsymmetrisch aufgebaute Bisphosphite im allgemeinen bei der Verwendung als Ligand in der übergangsmetallkatalysierten Hydroformylierung in der Flüssigphase deutlich geringere Reaktivitäten und geringere n-Regioselektivität als symmetrisch substituierte Bisphosphit-Liganden aufweisen; siehe in Rhodium-catalyzed Hydroformylation, ed. by P.W.N.M. van Leeuwen et C. Claver, Kluwer Academic Publishers 2006, AA Dordrecht, NL, Seite 45-46. Die in der erfindungsgemäßen Zusammensetzung verwendeten unsymmetrischen Bisphosphitliganden weisen die im Stand der Technik beschriebenen Nachteile nicht auf. Im Vergleich zu den im Stand der Technik genannten heterogenisierten symmetrischen Bisphosphiten weist die erfindungsgemäße Zusammensetzung die mit Abstand beste Katalysatorstandzeit auf und zeichnet sich damit durch hohe Stabilität aus.

Die erfindungsgemäßen Zusammensetzungen sowie deren Verwendung werden nachfolgend beispielhaft beschrieben, ohne dass die Erfindung auf diese beispielhaften Ausführungsformen beschränkt sein soll. Sind nachfolgende Bereiche, allgemeine Formeln oder Verbindungsklassen angegeben, so sollen diese nicht nur die entsprechenden Bereiche oder Gruppen von Verbindungen umfassen, die explizit erwähnt sind, sondern auch alle Teilbereiche und Teilgruppen von Verbindungen, die durch Herausnahme von einzelnen Werten (Bereichen) oder Verbindungen erhalten werden können. Werden im Rahmen der vorliegenden Beschreibung Dokumente zitiert, so soll deren Inhalt, insbesondere in Bezug auf den Sachverhalt, in dessen Zusammenhang das Dokument zitiert wurde, vollständig zum Offenbarungsgehalt der vorliegenden Erfindung gehören. Bei Prozentangaben handelt es sich, wenn nicht anders angegeben, um Angaben in Gewichtsprozent. Werden nachfolgend Mittelwerte angegeben, so handelt es sich, wenn nicht anderes angegeben, um Gewichtsmittel. Werden nachfolgend Parameter angegeben, die durch Messung bestimmt wurden, so wurden die Messungen, wenn nicht anders angegeben, bei einer Temperatur von 25 °C und einem Druck von 101.325 Pa durchgeführt.

Mit dem Begriff "inert" wird im Sinne der vorliegenden Erfindung die Eigenschaft von Stoffen, Komponenten, Lösungsmitteln oder Gemischen verstanden, welche sich dadurch auszeichnet, dass sich keine nachteiligen Auswirkungen oder dem beabsichtigten Reaktionsablauf gegenläufige Effekte einstellen.

Die erfindungsgemäße Zusammensetzung zeichnet sich dadurch aus, dass sie umfasst:
a) mindestens ein Trägermaterial, welches vorzugsweise porös ist;
b) mindestens eine Ionische Flüssigkeit;
c) mindestens ein Metall, ausgewählt aus der 9. Gruppe des Periodensystems der Elemente;
d) mindestens eine Verbindung der Formel (II) und
e) optional ein oder mehrere organische Amine.

Als Trägermaterialien können alle bekannten Trägermaterialien, vorzugsweise porösen Trägermaterialien eingesetzt werden. Vorzugsweise werden als poröse Trägermaterialien solche eingesetzt, die inert bezüglich der weiteren Bestandteile der Zusammensetzung und den Reaktionspartnern und -produkten der Reaktionen, bei denen die Zusammensetzungen eingesetzt werden. Bevorzugte Trägermaterialien sind anorganische, vorzugsweise oxidische Trägermaterialien. Als Trägermaterialien eigenen sich insbesondere Oxide von Aluminium, Silizium, Titan, Zirkon oder Aktivkohle oder Mischungen davon, die gegebenenfalls noch weitere Elemente aufweisen können. Bevorzugte Trägermaterialien sind z. B. Alumosilikate, Zeolithe, Al₂O₃ oder Siliziumdioxid. Besonders bevorzugt weist das Trägermaterial Siliziumdioxid auf oder besteht daraus. Das poröse Trägermaterial weist vorzugsweise einen oder mehrere der nachfolgenden Oberflächenparameter auf:
i) mittlerer Porendurchmesser in einem Bereich von 1 bis 423 nm;
ii) Porenvolumen in einem Bereich von 0,1 bis 2 ml/g;
iii) BET-Oberfläche in einem Bereich von 10 bis 2050 m²/g auf, wobei die Bestimmung dieser Werte nach der Hg-Methode gemäß DIN 66133 sowie der N₂-Adsorption nach DIN 66131 und DIN 66135 erfolgt. Bevorzugte poröse Trägermaterialien weisen alle der genannten Oberflächenparameter auf.

Im Rahmen der vorliegenden Erfindung werden unter Ionischen Flüssigkeiten wasserfreie [Gehalt an Wasser von weniger als 1,5 Gew.-% bezogen auf die Flüssigkeit] Flüssigkeiten verstanden, die bei einem Druck von 101.325 Pa und einer Temperatur von kleiner 373,15 K, vorzugsweise kleiner 323,15 K und besonders bevorzugt kleiner oder gleich 298,15 K im flüssigen Aggregatzustand vorliegen, und die zu mehr als 98 Gew.-% aus Ionen. Als IL können alle Ionischen Flüssigkeiten verwendet werden, die die vorgenannten Eigenschaften aufweisen. Bevorzugt werden als Ionische Flüssigkeit solche verwendet, bei denen das Anion ausgewählt ist aus der Gruppe umfassend:
Tetrafluoroborat ([BF₄]⁻), Hexafluorophosphat ([PF₆]⁻), Dicyanamid ([N(CN)₂]⁻), Bis(trifluoromethylsulfonyl)imid ([NTf₂]⁻), Tricyanomethid ([C(CN)₃]⁻), Tetracyanoborat ([B(CN)₄]⁻), Halogenide (Cl⁻, Br⁻, F⁻, I⁻), Hexafluoroantimonat ([SbF₆]⁻), Hexafluoroarsenat ([AsF₆]⁻), Sulfat ([SO₄]²⁻), Tosylat ([C₇H₇SO₃]⁻), Triflat (CF₃SO₃⁻), Nonaflat ([C₄F₉SO₃-), Tris-(Pentafluoroethyl)-trifluorophosphat ([PF₃(C₂F₅)₃]-), Thiocyanat ([SCN]⁻), Carbonat ([CO₃]²⁻), [R^{A}-COO]⁻, [R^{A}-SO₃]⁻, [R^{A}-SO₄]⁻, [R^{A}PO₄R^{B}]⁻ oder [(R^{A}-SO₂)₂N]⁻ mit R^{A} und R^{B} gleich oder ungleich, jeweils ein linearer oder verzweigter 1 bis 12 Kohlenstoffatome enthaltender aliphatischer oder alicyclischer Alkyl- oder Perfluoralkyl- oder ein C₅-C₁₈-substituierter Aryl-, C₅-C₁₈- substituierter Aryl-C₁-C₆-alkyl- oder C₁-C₆-Alkyl-C₅-C₁₈-substituierter Aryl-Rest ist, der durch Halogenatome substituiert ein kann;
und das Kation ausgewählt ist aus der Gruppe umfassend:
   quaternäre Ammonium-Kationen der allgemeinen Formel [NR₁R₂R₃R₄]⁺, mit R₁, R₂, R₃, R₄ ausgewählt aus C₁-C₈-Alkylgruppen;
   Phosphonium-Kationen der allgemeinen Formel [PR₁R₂R₃R₄]⁺ mit R₁, R₂, R₃, R₄ ausgewählt aus C₁-C₈-Alkylgruppen,
   Imidazolium-Kationen der allgemeinen Formel (IV) 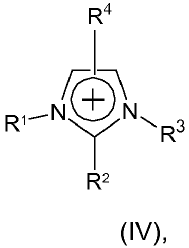 wobei der Imidazol-Kern substituiert sein kann mit wenigstens einer Gruppe R, die ausgewählt ist aus C₁-C₈-Alkyl-, C₁-C₆-Alkoxy-, C₁-C₆- substituierte Aminoalkyl-, C₅-C₁₂-substituierte Aryl- oder C₅-C₁₂- substituierte Aryl-C₁-C₆-Alkylgruppen,
   Pyridinium-Kationen der allgemeinen Formel (V) 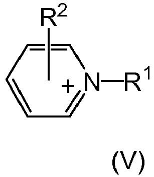 wobei der Pyridin-Kern substituiert sein kann mit wenigstens einer Gruppe R, die ausgewählt ist aus C₁-C₆-Alkyl-, C₁-C₆-Alkoxy-, C₁-C₆- substituierte Aminoalkyl-, C₅-C₁₂-substituierte Aryl- oder C₅-C₁₂- substituierte Aryl-C₁-C₆-Alkylgruppen,
   Pyrazolium-Kationen der allgemeinen Formel (VI) 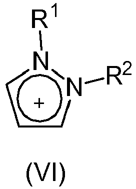 wobei der Pyrazol-Kern substituiert sein kann mit wenigstens einer Gruppe R, die ausgewählt ist aus C₁-C₆-Alkyl-, C₁-C₆-Alkoxy-, C₁-C₆- substituierte Aminoalkyl-, C₅-C₁₂-substituierte Aryl- oder C₅-C₁₂- substituierte Aryl-C₁-C₆-Alkylgruppen;
   Triazolium-Kationen der allgemeinen Formel (VII) 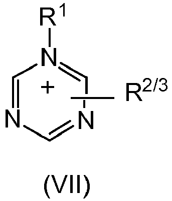 wobei der Triazol-Kern substituiert sein kann mit wenigstens einer Gruppe R, die ausgewählt ist aus C₁-C₆-Alkyl-, C₁-C₆-Alkoxy-, C₁-C₆- substituierte Aminoalkyl-, C₅-C₁₂-substituierte Aryl- oder C₅-C₁₂- substituierte Aryl-C₁-C₆-Alkylgruppen.

Bevorzugte Ionische Flüssigkeiten sind solche, die als Kation eine Imidazolium-Struktur der allgemeinen Formel (IV) aufweisen, 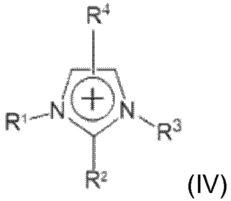 wobei der Imidazol-Kern mit wenigstens einer Gruppe R substituiert ist, ausgewählt unter C₁ - C₈-Alkylgruppen.

Besonders bevorzugt in der erfindungsgemäßen Zusammensetzung ist die Ionische Flüssigkeit ausgewählt aus der Gruppe umfassend:
a) 1-Ethyl-3-methylimidazolium bis(trifluoromethylsulfonyl)imid,
b) 1-Butyl-3-methylimidazolium hexafluorophosphat,
c) 1-Butyl-3-methylimidazolium tetrafluoroborat,
d) 1-Butyl-3-methylimidazolium bis(trifluoromethylsulfonyl)imid,
e) 1-Ethyl-3-methylimidazolium ethylsulfat,
f) Trioctyl-Methylammonium bis(trifluoromethylsulfonyl)imid,
g) 1-Butyl-3-methylimidazolium octylsulfat. Die Verbindung der Formel (II) in der erfindungsgemäßen Zusammensetzung ist: 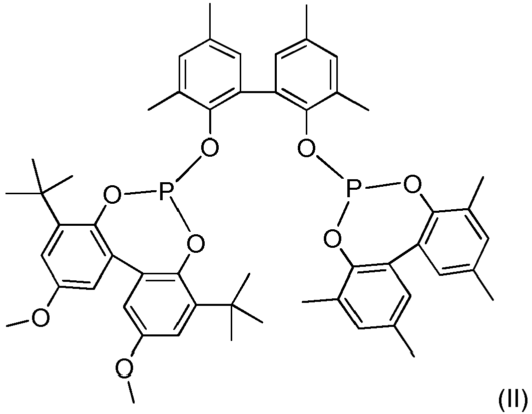 wobei die Verbindung der Formel (II) weitere Bestandteile aufweisen kann, welche auf Verunreinigungen in der Synthese von (II), wie z.B. unumgesetztes Edukt, Hydrolyse-, Oxidationsprodukte von (II) oder weitere Verunreinigungen, zurückzuführen sind. Vorzugsweise weist die erfindungsgemäße Zusammensetzung ein organisches Amin auf. Bevorzugt umfasst das verwendete organische Amin mindestens einen Rest mit einer 2,2,6,6-Tetramethylpiperidineinheit nach Formel (III): 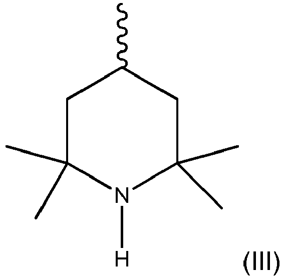 Besonders bevorzugt ist das organische Amin in der erfindungsgemäßen Zusammensetzung ausgewählt aus den Verbindungen der Formeln (IIIa) - (IIIh): 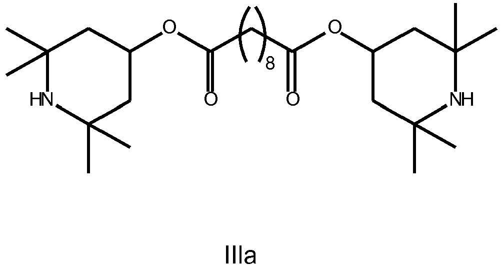 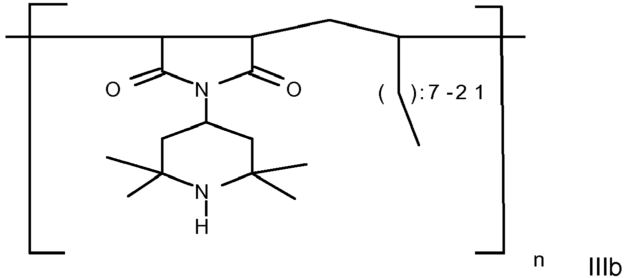 mit n = 1 bis 20 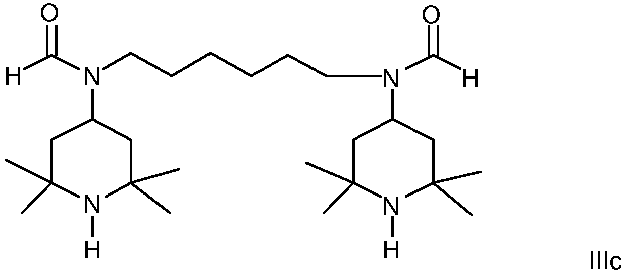 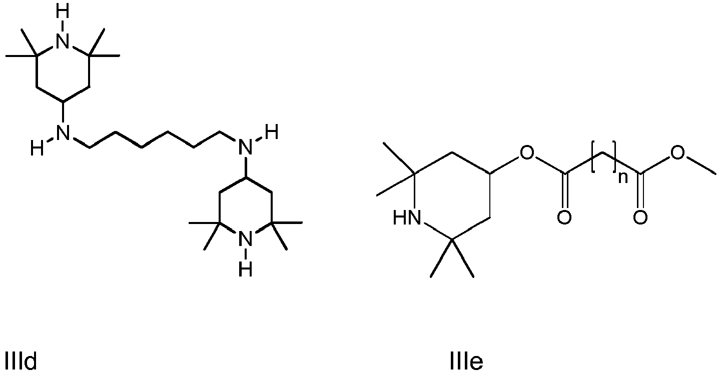 mit n = 1 bis 12 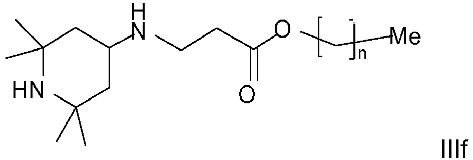 mit n = 1 bis 17 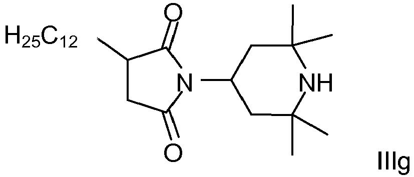 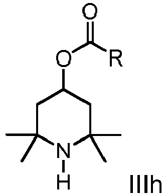 mit R = C₆- bis C₂₀-Alkyl

In der erfindungsgemäßen Zusammensetzung ist das Metall vorzugsweise ausgewählt unter:
Cobalt, Rhodium, Iridium, insbesondere Rhodium.

Die erfindungsgemäße Zusammensetzung kann auf jede bekannte Weise durch Mischen der Komponenten hergestellt werden. Vorzugsweise wird die erfindungsgemäße Zusammensetzung durch das nachfolgend beschriebene erfindungsgemäße Verfahren hergestellt bzw. ist dadurch erhältlich.

Das erfindungsgemäße Verfahren zur Herstellung der erfindungsgemäßen Zusammensetzung, zeichnet sich dadurch aus, dass es die Schritte aufweist:
A) vorlegen einer Vorstufe mindestens einer Verbindung eines Metalls aus der 9. Gruppe des Periodensystems der Elemente, vorteilhafterweise Rhodium;
B) in-Kontakt-bringen mindestens einer Verbindung eines Metalls aus der 9. Gruppe des Periodensystems der Elemente, vorteilhafterweise Rhodium, mit einem molaren Überschuss an mindestens einer Verbindung der Formel (II): 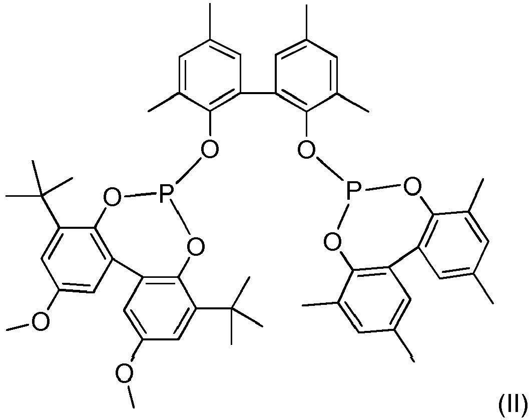
wobei die Verbindung der Formel (II) weitere Bestandteile aufweisen kann, welche auf Verunreinigungen in der Synthese von (II), wie z.B. unumgesetztes Edukt, Hydrolyse-, Oxidationsprodukte von (II) oder weitere Verunreinigungen, zurückzuführen sind ; mindestens einer Ionischen Flüssigkeit, wobei bevorzugt als Ionische Flüssigkeit solche verwendet werden, bei denen das Anion ausgewählt ist aus der Gruppe umfassend: Tetrafluoroborat ([BF₄]⁻), Hexafluorophosphat ([PF₆]⁻), Dicyanamid ([N(CN)₂]⁻), Bis(trifluoromethylsulfonyl)imid ([NTf₂]⁻), Tricyanomethid ([C(CN)₃]⁻), Tetracyanoborat ([B(CN)₄]⁻), Halogenide (Cl⁻, Br⁻, F⁻, I⁻), Hexafluoroantimonat ([SbF₆]⁻), Hexafluoroarsenat ([AsF₆]⁻), Sulfat ([SO₄]²⁻), Tosylat ([C₇H₇SO₃]⁻), Triflat (CF₃SO₃⁻), Nonaflat ([C₄F₉SO₃⁻), Tris-(Pentafluoroethyl)-trifluorophosphat ([PF₃(C₂F₅)₃]-), Thiocyanat ([SCN]⁻), Carbonat ([CO₃]²⁻), [R^{A}-COO]⁻, [R^{A}-SO₃]⁻, [R^{A}-SO₄]⁻, [R^{A}PO₄R^{B}]⁻ oder [(R^{A}-SO₂)₂N]⁻ mit R^{A} und R^{B} gleich oder ungleich, jeweils ein linearer oder verzweigter 1 bis 12 Kohlenstoffatome enthaltender aliphatischer oder alicyclischer Alkyl- oder Perfluoralkyl- oder ein C₅-C₁₈-substituierter Aryl-, C₅-C₁₈- substituierter Aryl-C₁-C₆-alkyl- oder C₁-C₆-Alkyl-C₅-C₁₈-substituierter Aryl-Rest ist, der durch Halogenatome substituiert ein kann; und das Kation ausgewählt ist aus der Gruppe umfassend:
quaternäre Ammonium-Kationen der allgemeinen Formel [NR₁R₂R₃R₄]⁺ mit R₁, R₂, R₃, R₄ ausgewählt aus C₁-C₈-Alkylgruppen;
Phosphonium-Kationen der allgemeinen Formel [PR₁R₂R₃R₄]⁺ mit R₁, R₂, R₃, R₄ ausgewählt aus C₁-C₈-Alkylgruppen;
Imidazolium-Kationen der allgemeinen Formel (IV) 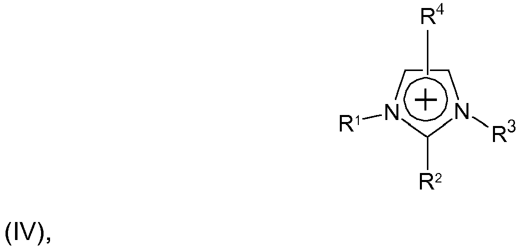 wobei der Imidazol-Kern substituiert sein kann mit wenigstens einer Gruppe R, die ausgewählt ist aus C₁-C₈-Alkyl-, C₁-C₆-Alkoxy-, C₁-C₆- substituierte Aminoalkyl-, C₅-C₁₂-substituierte Aryl- oder C₅-C₁₂- substituierte Aryl-C₁-C₆-Alkylgruppen,
Pyridinium-Kationen der allgemeinen Formel (V) 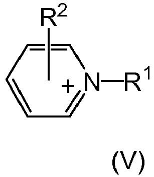 wobei der Pyridin-Kern substituiert sein kann mit wenigstens einer Gruppe R, die ausgewählt ist aus C₁-C₆-Alkyl-, C₁-C₆-Alkoxy-, C₁-C₆- substituierte Aminoalkyl-, C₅-C₁₂-substituierte Aryl- oder C₅-C₁₂- substituierte Aryl-C₁-C₆-Alkylgruppen,
Pyrazolium-Kationen der allgemeinen Formel (VI) 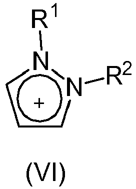 wobei der Pyrazol-Kern substituiert sein kann mit wenigstens einer Gruppe R, die ausgewählt ist aus C₁-C₆-Alkyl-, C₁-C₆-Alkoxy-, C₁-C₆- substituierte Aminoalkyl-, C₅-C₁₂-substituierte Aryl- oder C₅-C₁₂- substituierte Aryl-C₁-C₆-Alkylgruppen,
Triazolium-Kationen der allgemeinen Formel (VII) 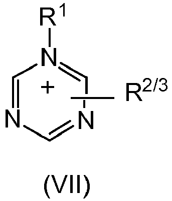 wobei der Triazol-Kern substituiert sein kann mit wenigstens einer Gruppe R, die ausgewählt ist aus C₁-C₆-Alkyl-, C₁-C₆-Alkoxy-, C₁-C₆- substituierte Aminoalkyl-, C₅-C₁₂-substituierte Aryl- oder C₅-C₁₂- substituierte Aryl-C₁-C₆-Alkylgruppen,
bevorzugte Ionische Flüssigkeiten sind solche, die als Kation eine Imidazolium-Struktur der allgemeinen Formel (IV) aufweisen, 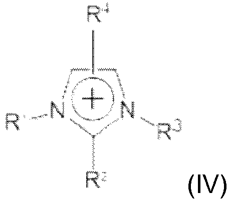 wobei der Imidazol-Kern mit wenigstens einer Gruppe R substituiert ist, ausgewählt unter C₁ - C₈-Alkylgruppen;
besonders bevorzugt in der erfindungsgemäßen Zusammensetzung ist die Ionische Flüssigkeit ausgewählt aus der Gruppe umfassend:
   a) 1-Ethyl-3-methylimidazolium bis(trifluoromethylsulfonyl)imid,
   b) 1-Butyl-3-methylimidazolium hexafluorophosphat,
   c) 1-Butyl-3-methylimidazolium tetrafluoroborat,
   d) 1-Butyl-3-methylimidazolium bis(trifluoromethylsulfonyl)imid,
   e) 1-Ethyl-3-methylimidazolium ethylsulfat,
   f) Trioctyl-Methylammonium bis(trifluoromethylsulfonyl)imid,
   g) 1-Butyl-3-methylimidazolium octylsulfat und optional ein oder mehrere organische Amine, wobei vorteilhafterweise das verwendete organische Amin mindestens einen Rest mit einer 2,2,6,6-Tetramethylpiperidineinheit nach Formel (III): 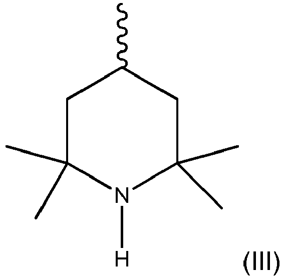 aufweist; besonders bevorzugt ist das organische Amin in der erfindungsgemäßen Zusammensetzung ausgewählt aus den Verbindungen der Formeln (IIIa) - (IIIh): 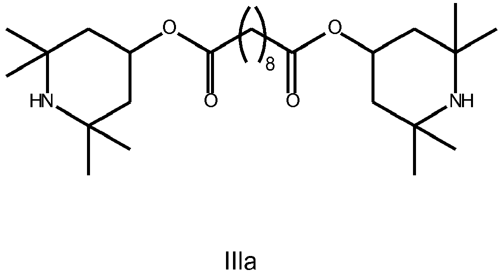 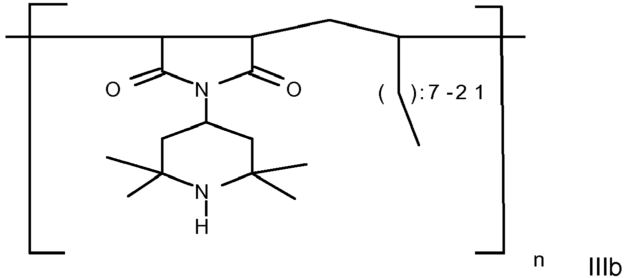 mit n = 1 bis 20 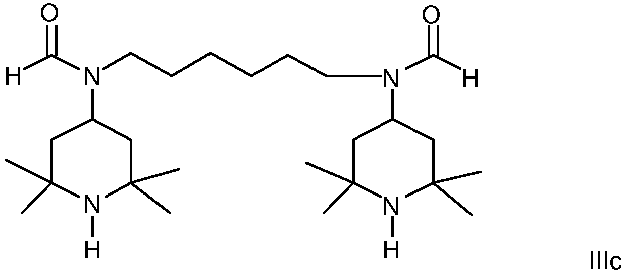 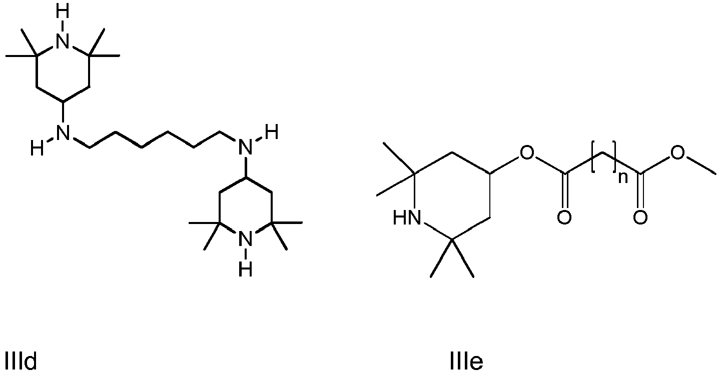 mit n = 1 bis 12 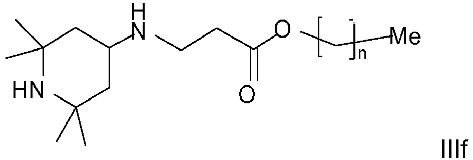 mit n = 1 bis 17 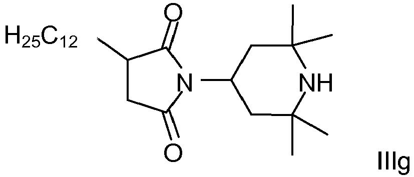 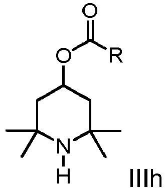
   mit R = C₆- bis C₂₀-Alkyl; unter Verwendung eines inerten Lösungsmittels; C) Zugabe mindestens eines porösen, inerten Trägermaterials zu der unter b) generierten Mischung, wobei bevorzugte poröse, inerte Trägermaterialen beispielsweise Alumosilikate, Zeolithe, Al₂O₃ oder Siliziumdioxid sind, insbesondere bevorzugt ist Siliziumdioxid und wobei bevorzugte poröse, inerte Trägermaterialien einen oder mehrere der nachfolgenden Oberflächenparameter aufweisen: i) mittlerer Porendurchmesser in einem Bereich von 1 bis 423 nm; ii) Porenvolumen in einem Bereich von 0,1 bis 2 ml/g; iii) BET-Oberfläche in einem Bereich von 10 bis 2050 m²/g auf, wobei die Bestimmung dieser Werte nach der Hg-Methode gemäß DIN 66133 sowie der N₂-Adsorption nach DIN 66131 und DIN 66135 erfolgt; besonders bevorzugte poröse Trägermaterialien weisen alle der genannten Oberflächenparameter auf; D) entfernen des inerten Lösungsmittels unter Erhalt der katalytisch aktiven Zusammensetzung;
   wobei bevorzugt die Schritte A) bis C) in beliebiger Reihenfolge durchgeführt und wobei bevorzugt in Schritt A) mindestens eine Verbindung eines Metalls aus der 9. Gruppe des Periodensystems der Elemente, vorteilhafterweise Rhodium, in einem inerten Lösungsmittel vorgelegt werden kann. Ein vorteilhaftes inertes Lösungsmittel im Sinne der vorliegenden Erfindung ist beispielsweise Dichlormethan.

Die oben beschriebene erfindungsgemäße Zusammensetzung kann als katalytisch aktive Zusammensetzung eingesetzt werden. Vorzugsweise wird die erfindungsgemäße Zusammensetzung als katalytisch aktive Zusammensetzung in einem Verfahren zur Hydroformylierung von ungesättigten Verbindungen oder Gemischen davon verwendet. Das erfindungsgemäße Verfahren zur Hydroformylierung von ungesättigten Verbindungen zeichnet sich demgemäß dadurch aus, dass eine erfindungsgemäße Zusammensetzung als Katalysator eingesetzt wird.

Die ungesättigten Verbindungen sind vorzugsweise ausgewählt aus C₂ - C₄ - Olefinen und deren technischen Gemischen, wie sie z.B. als Raffinatströme - Raffinat I, II, oder III - bei der Auf- und Weiterverarbeitung in der petrochemischen Industrie vorliegen, insbesondere Ethen, Propen, Butene oder Gemische, die diese Verbindungen aufweisen.

Es ist vorteilhaft, wenn das erfindungsgemäße Verfahren zur Hydroformylierung von ungesättigten Verbindungen unter Verwendung der erfindungsgemäßen Zusammensetzung als katalytisch aktive Zusammensetzung in heterogenisierter Form, beispielsweise in einem Festbettreaktor, ausgeführt wird. Vorteilhafterweise wird das erfindungsgemäße Verfahren in einem Temperaturbereich von 353,15 K bis 433,15 K, bevorzugt zwischen 363,15 K bis 413,15 K sowie einem Druckbereich von 0,5 bis 1,1 MPa durchgeführt. Es ist vorteilhaft, wenn das molare Verhältnis von H₂ zu 1-Buten zwischen 1:1 bis 15:1, insbesondere zwischen 6:1 bis 8:1 unter den Reaktionsbedingungen beträgt. In dem erfindungsgemäßen Verfahren erfolgt die Abtrennung der Reaktionsprodukte vorzugsweise über die Gasphase, wobei Gasgemische umfassend CO und H₂ als Strip-Gas verwendet werden.

Abschließender Gegenstand der vorliegenden Erfindung ist ein mehrphasiges Reaktionsgemisch, beinhaltend:
1) mindestens eine ungesättigte Verbindung;
2) ein Gasgemisch, umfassend Kohlenmonoxid, Wasserstoff sowie
3) Aldehyde und ihre Folgeprodukte, in Gegenwart der zuvor beschriebenen erfindungsgemäßen Zusammensetzung. Vorzugsweise handelt es sich bei den ungesättigten Verbindungen um C₂ - C₄-Olefine und/oder deren technischen Gemischen, die C₂ - C₄-Olefine aufweisen können.

Die vorliegende Erfindung wird durch die Figuren 1, 2 und 3 näher erläutert ohne darauf beschränkt zu sein.
Figur 1 stellt das Katalyseexperiment einer Hydroformylierung von C4-Gemischen gemäß DE 10 2013217174 als 1. Vergleichsbeispiel dar, wobei die Hydroformylierung an einer katalytisch aktiven Zusammensetzung bestehend aus einem inerten porösen Trägermaterial durchgeführt wird ohne Verwendung einer Ionischen Flüssigkeit und ohne Verwendung eines organischen Amins.
Figur 2 zeigt das Katalyseexperiment unter Verwendung der erfindungsgemäßen Zusammensetzung als katalytisch aktive Zusammensetzung in einer Hydroformylierung von C4-Gemischen als erfindungsgemäßes Beispiel.
Figur 3 stellt direkt die Ergebnisse der Katalyseexperimente unter Verwendung der erfindungsgemäßen Zusammensetzung als katalytisch aktive Zusammensetzung in einer Hydroformylierung von C4-Gemischen denen des 2. Vergleichsbeispiels gegenüber.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

### Beispiele:

Alle nachfolgenden Präparationen wurden mit Standard-Schlenk-Technik unter Schutzgas durchgeführt. Die Lösungsmittel wurden vor Gebrauch, falls nicht anders vermerkt, über geeigneten Trocknungsmitteln getrocknet (Purification of Laboratory Chemicals, W. L. F. Armarego (Autor), Christina Chai (Autor), Butterworth Heinemann (Elsevier), 6. Auflage, Oxford 2009).

### Chemikalien

(Acetylacetonato)dicarbonylrhodium- in Kurzform (Rh(acac)(CO)₂) - und Dichlormethan (HPLC Reinheit) wurden ohne weitere Aufreinigung verwendet. Das mittelporige Siliziumdioxid ist kommerziell als Kieselgel 100 (0,2-0,5 mm) für die Säulen-Chromatographie bei der Firma Merck KGaA erhältlich. Siliziumdioxid wurde zur Herstellung der katalytisch aktiven Zusammensetzung 24 h bei 450°C kalziniert und anschließend weitere 24 h unter Vakuum bei 200 Pa gelagert. Weitere Lagerung des Siliziumdioxides erfolgte unter Argonathmosphäre. Der Ligand (II) wurde gemäß nachfolgenden Reaktionsschema hergestellt:

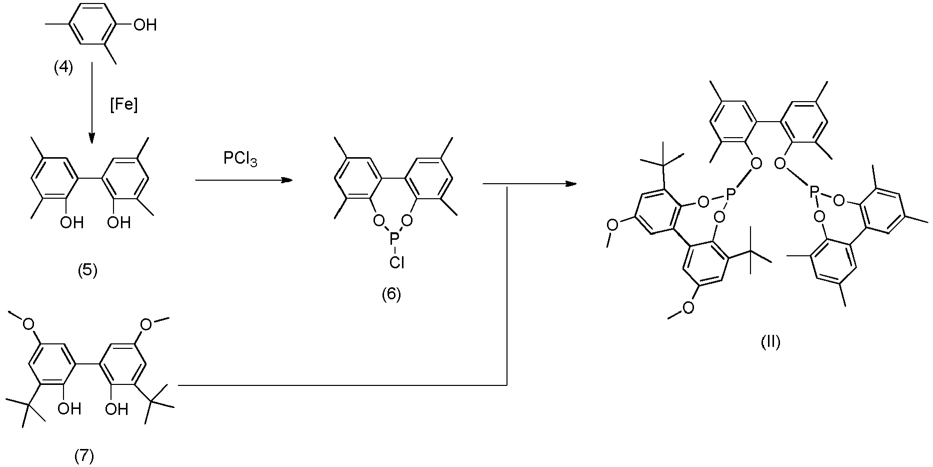

### Abkürzungen:

VE-Wasser = demineralisiertes Wasser
KPG = Kerngezogenes Präzisions-Glasgerät
ACN = Acetonitril
acac = acetylacetonat
NEt₃ = Triethylamin

Alle nachfolgenden Präparationen wurden mit Standard-Schlenk-Technik unter Schutzgas durchgeführt. Die Lösungsmittel wurden vor Gebrauch über geeigneten Trocknungsmitteln getrocknet (Purification of Laboratory Chemicals, W. L. F. Armarego (Autor), Christina Chai (Autor), Butterworth Heinemann (Elsevier), 6. Auflage, Oxford 2009).

Die Charakterisierung der Produkte erfolgte mittels NMR-Spektroskopie. Chemische Verschiebungen (δ) werden in ppm angegeben. Die Referenzierung der ³¹P-NMR-Signale erfolgte gemäß: SR_{31P} = SR_{1H} * (BF31P / BF_{1H}) - SR_{1H} * 0,4048. (Robin K. Harris, Edwin D. Becker, Sonia M. Cabral de Menezes, Robin Goodfellow, and Pierre Granger, Pure Appl. Chem., 2001, 73, 1795-1818; Robin K. Harris, Edwin D. Becker, Sonia M. Cabral de Menezes, Pierre Granger, Roy E. Hoffman and Kurt W. Zilm, Pure Appl. Chem., 2008, 80, 59-84). Mittels der ³¹P-NMR wurde der Gehalt des Liganden (II) bestimmt, wobei dieser unsymmetrische Ligand durch zwei Phosphorsignale charakterisiert wird.

### Synthese des 2,2'-Bis-(3,5-dimethylphenol)chlorophosphits (6)

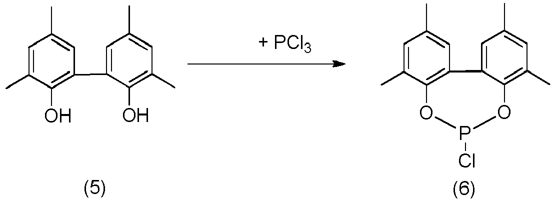

In einem sekurierten 2 L Schlenk mit Magnetrührer wurden 440 ml Phosphortrichlorid vorgelegt. In einem zweiten sekurierten 1 L Schlenk wurden 120 g 2,2' -Bis-(3,5-dimethylphenol) eingewogen und unter Rühren 500 ml getrocknetes Toluol hinzugefügt. Die Biphenol-Toluol-Suspension wurde innerhalb von 4 h bei 63°C zum Phosphortrich-Iorid dosiert. Nach vollständiger Zugabe wurde die Reaktionsmischung über Nacht bei Temperatur gerührt. Am nächsten Morgen wurde die Lösung in der Wärme (45°C) eingeengt und das Produkt konnten in 96,5%iger Ausbeute (153 g) erhalten werden.
³¹P-NMR: 175,59 (94,8% 2,2'-Bis-(3,5-dimethylphenol)chlorophosphit), 4,4% diverse PCI-Verbindungen, 0,8% P-H-Verbindung.

### Synthesevorschrift zur Herstellung des reinen Liganden (II)

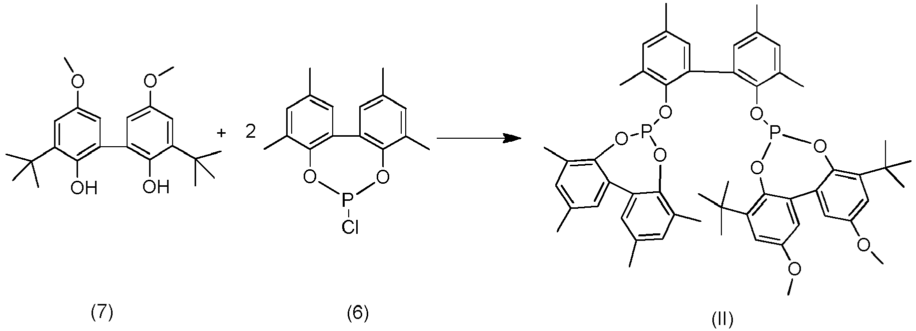

In einem 1000 ml Schlenk wurde unter Schutzgas 38,75 g (0,121 mol) 2,2'-Bis-(3,5-dimethylphenyl)chlorophosphit in 150 ml entgastem ACN gelöst und auf 35°C erwärmt. In einem zweiten Schlenk (500 ml) wurden 20,1 g (0,056 mol) 3,3'-Di-tert.-butyl-5,5'-dimethoxy-[1,1'-biphenyl]-2,2'diol in 150 ml entgastem ACN gelöst und unter Rühren mit 40,9 ml entgastem NEt₃ (0,29 mol) versetzt. Dann wurde langsam die Biphenol/ NEt₃-Lösung zu der Chlorophosphitlösung getropft. Nach einer Nachreaktionszeit von 1 h wurde die Reaktionslösung über Nacht bei 45°C gerührt.

Dieser Feststoff wurde in entgastem ACN 1.5h bei 75°C suspendiert und abgetrennt und mit warmen ACN nachgewaschen. Anschließend wurde das Produkt in Toluol 1.5h bei 35°C suspendiert und nachgewaschen. Das Zielprodukt (II) konnte als weißer Feststoff (33 g, 66%) erhalten werden. ³¹P-NMR (202,4 MHz, toluene-d₈): 142,5 und 140,9 (100%)

### Herstellung des Liganden VIII für 2. Vergleichsbeispiel

Die Präparation der katalytisch aktiven Zusammensetzung mit dem Liganden (VIII) erfolgt in Anlehnung an DE 102010041821.

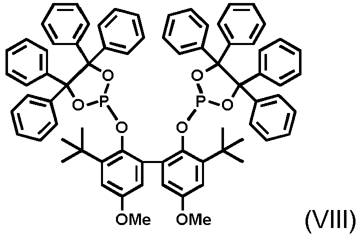

### Herstellung der erfindungsgemäßen katalytisch aktiven Zusammensetzung Rh-(II)

Siliziumdioxid als inertes, poröses Trägermaterial wird zur Kalzinierung bzw. thermischen Vorbehandlung über 24 h auf 450°C erhitzt woran sich weitere 24h unter Vakuum bei 200 Pa anschließen. Daraufhin wird das Siliziumdioxid unter einer ArgonAtmosphäre gelagert. 0,052 g bzw. 0,2 mmol Rhodium Dicarbonylacetylacetonat - in Kurzform Rh(acac)(CO)₂ - werden in ca. 50 ml CH₂Cl₂ gelöst und für 10 min gerührt. Anschließend werden 2 mmol des jeweils benutzten Liganden der Formel (II) unter Rühren zugegeben. Nach weiteren 10 Minuten werden 8 mmol des organischen Amins Bis(2,2,6,6-tetramethyl-4-piperidyl)sebacat (IIIa) - in Kurzform OA - zugegeben. Die Zugabe der Ionischen Flüssigkeit IL - z. B. 1-Ethyl-3-methylimidazolium-bis(trifluoromethansulfonyl)imid, in Kurzform [EMIM][NTf₂] - erfolgt nach weiteren 10 min rühren, wobei ein Beladungsgrad α derart eingestellt wird, dass er einen Wert von 0,1 bzw. 10 Vol.-% annimmt. Unter einem Beladungsgrad α versteht man im Zusammenhang mit SILP-Katalysatorsystemen das Verhältnis von dem Volumen der jeweils verwendeten Ionischen Flüssigkeit IL zu dem Porenvolumen des jeweils verwendeten Trägermaterials. Der zuvor angegebene Wert des Beladungsgrads α von 0,1 bzw. 10 Vol.-% ist aus Vorversuchen ermittelt worden. Er stellt ein Optimum hinsichtlich der katalytischen Aktivität - typischerweise angegeben als TOF bzw. Turn-Over-Frequency in h⁻¹ - und des Rückhalts der jeweils verwendeten übergangsmetallhaltigen Komplexverbindungen auf dem inerten, porösen Trägermaterial dar. Nach weiteren 30 min, werden 10 g kalziniertes Siliziumdioxid (Silica 100, Merck) zugegeben und damit eine Rhodium-Beladung des SILP-Katalysatorsystems m_{Rh} von 0,2 Massen-% eingestellt. Nach ca. 60 min wird das Lösemittel vorsichtig am Rotationsverdampfer entfernt und anschließend bis zum Gebrauch unter Argon gelagert.

### Katalyse-Experimente

Sämtliche Hydroformylierungsversuche wurden in einem Festbettreaktor durchgeführt. Das trockene Katalysatormaterial wurde in den Rohrreaktor gefüllt und von beiden Seiten mit einem Stück Glaswolle fixiert. Die gesamt Anlage wurde bei Raumtemperatur mit Argon gespült und anschließend mit dem Reaktionsdruck (Argon) beaufschlagt. Falls kein Druckverlust festzustellen war, wurde der Reaktor in Argonstrom auf Reaktionstemperatur aufgeheizt. Nach Einstellen der jeweiligen Volumenströme, beispielsweise 100 Nml/min, wird für 4 Stunden bei 100°C und 1,0 MPa Synthesegas (CO und H₂; Volumen = 1 : 1, ≥ 99,97%) durch den Reaktor geleitet und das SILP-Katalysatorsystem präformiert.

Die Zudosierung des Synthesegases erfolgt durch einen Massendurchflussregler (Bezugsquelle Fa. Bronkhorst). Die Eduktdosierung der C4-Mischung erfolgte über eine HPLC-Pumpe (Bezugsquelle Fa. Knauer). In einem mit Glasperlen gefüllten Mischer wurde der Eduktgasstrom homogenisiert bevor dieser den Rohrreaktor samt Katalysatorschüttung von oben her durchströmte. Der Reaktor bestand aus rostfreiem Edelstahl (Durchmesser 12 mm, Länge 390 mm) und besaß an der Ausgangseite eine Gitter zur Positionierung des Katalysatormaterials. Durch ein innen liegendes Thermoelement konnte die Temperatur in der Katalysatorschüttung aufgezeichnet werden. Der Gesamtdruck in der Versuchsanlage wurde über ein elektronisches Druckhalteventil (Bezugsquelle Fa. Samson) geregelt. Auf der Niederdruckseite wurde der Produktgasstrom mit Hilfe eines Ventils aufgeteilt, sodass nur ein kleiner Anteil des Gesamtstromes zum Online-Gaschromatographen (Bezugsquelle Fa. Agilent, Modell 6890) geleitet wurde. Der größere Anteil wurde direkt in ein Produktfass geleitet. Durch ein Ventil wurden in regelmäßigen Zeitabständen Proben des Produktgasstroms in den Gaschromatographen injiziert. Die Datenauswertung erfolgte durch die ChemStation Software aus der Fa. Agilent.

### Analytik

Die Produktgaszusammensetzung während der Versuchslaufzeit wurde mit einem Online-Gaschromatographen analysiert. Der Gaschromatograph war ausgestattet mit einer Dimethylpolysiloxan beschichteten Säule (Fa. Agilent Technologies, Länge 50 m, Innendurchmesser 0,2 mm, Filmdicke 0,5 µm) und einem Flammenionisationsdetektor (FID). Eingestellte Messparameter: Injektortemperatur 423,15 K, Split-Verhältnis 33,5:1, konstanter Säulenfluss Helium 74 ml min⁻¹, Detektortemperatur 523,15 K, Heizrampe: Anfangstemperatur 323,15 K, Haltezeit 15 min, Heizen auf 473,15 K mit 25 K min⁻¹, Haltezeit 40 min, Gesamtzeit pro Messung 61 min.

### Gaszusammensetzungen der Katalyseexperimente

**Tabelle 1:**

| | Zusammensetzung C4-Gemisch 2. Vergleichsbeispiel / %^{[a]} Rh-(VIII) | Zusammensetzung C4-Gemisch erfindungsgemäßes Beispiel / %^{[a]} Rh-(II) |
|---|---|---|
| 1-Buten + iso-Buten | 27,6 | 27,4 |
| | (27,5+< 0,1) | (27,3+< 0,1) |
| Cis-2-Buten | 16 | 15 |
| Trans-2-Buten | 28 | 25 |
| n-Butan | 28,3 | 29,5 |
| Iso-Butan | < 0,1 | < 0,1 |
| 2-Methylbutan | | 3 |

| | | |
|---|---|---|
| [a] GC-Fläche in % (Säule Fa. Agilent Technologies Länge 50 m, Innendurchmesser 0,32 mm, Filmdicke 0,5 µm, Trägergas Helium; Detektor: FID, Verdampfertemperatur 473,15 K, Split-Verhältnis 33,5:1, konstanter Säulenfluss Helium 91,6 ml min⁻¹, Detektortemperatur 523,15 K, Heizrampe: Anfangstemperatur 323,15 K, Haltezeit 15 min, Heizen auf 473,15 K mit 25 K min⁻¹, Haltezeit 40 min, Gesamtzeit pro Messung 61 min) | | |

### 1. Vergleichsbeispiel gemäß DE 10 2013217174:

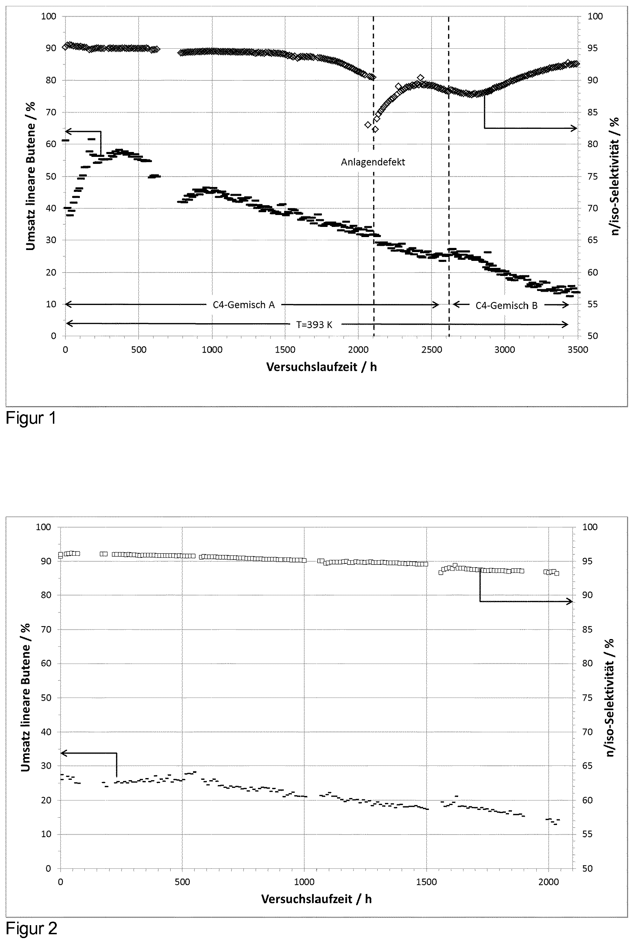

In Figur 1 ist ein Katalyseexperiment zur Hydroformylierung von C4-Gemischen (Zusammensetzung siehe Tabelle 2) gezeigt.

**Tabelle 2:**

| | Zusammensetzung C4-Gemisch A 1. Vergleichsbeispiel / %^{[a]} Rh-(II): 0 - 2615 h | Zusammensetzung C4-Gemisch B 1. Vergleichsbeispiel/ %^{[a]} Rh-(II): 2615 - 3506 h |
|---|---|---|
| 1-Buten + iso-Buten | 24,9 | 27 |
| | (24,8 + < 0,1) | (26,9+< 0,1) |
| Cis-2-Buten | 16 | 16 |
| Trans-2-Buten | 33 | 29 |
| n-Butan | 26 | 27,9 |
| Iso-Butan | < 0,1 | < 0,1 |

| | | |
|---|---|---|
| [a] GC-Fläche in % (Säule Fa. Agilent Technologies Länge 50 m, Innendurchmesser 0,32 mm, Filmdicke 0,5 µm, Trägergas Helium; Detektor: FID, Verdampfertemperatur 473,15 K, Split-Verhältnis 33,5:1, konstanter Säulenfluss Helium 91,6 ml min⁻¹, Detektortemperatur 523,15 K, Heizrampe: Anfangstemperatur 323,15 K, Haltezeit 15 min, Heizen auf 473,15 K mit 25 K min⁻¹, Haltezeit 40 min, Gesamtzeit pro Messung 61 min) | | |

In Figur 1 ist mit - der Umsatz der linearen Butene und mit ◇ die n/iso-Selektivität über die Versuchslaufzeit gezeigt. Für das Katalyseexperiment wurden 12 g der katalytisch aktiven Verbindung Rh-(II) ohne organisches Amin oder ionische Flüssigkeit eingesetzt. Als Trägermaterial wurde Kieselgel 100 (0,2-0,5 mm) für die Säulen-Chromatographie der Firma Merck KGaA verwendet. Der Metallgehalt der katalytisch aktiven Verbindung beträgt 0,2 Gew-% bezogen auf das Trägermaterial. Das molare Verhältnis von Ligand (II) zu Rhodium ist 10 : 1. Der Versuch wurde bei einer Reaktionstemperatur von 393,15K und einem Druck von 1 MPa; (p(1- und 2-Butene)=0,16 MPa; p(Butane)=0,06 MPa; p(H₂)=p(CO)=0,39 MPa, molares H₂/1-Buten-Verhältnis 6 : 1,Verweilzeit=58 s) durchgeführt. Zur besseren Übersichtlichkeit wurden von den ermittelten Messwerten nur jeder 10te gezeigt. Bei 2106 h trat ein kurzzeitiger Anlagenausfall aufgrund Defekts in der Anlage ohne Einbruch von Luftsauerstoff auf. Die Analytik und die Durchführung des Katalyseexperiments ist analog zum erfindungsgemäßen Beispiel.

### Herstellung der katalytisch aktiven Zusammensetzung für 1. Vergleichsbeispiel:

Sämtliche Präparationen der katalytisch aktiven Zusammensetzung erfolgten mittels Schlenktechnik unter Argon (≥ 99.99 %). 0,40 mmol Rh(CO)2(acac) wurde in ca. 160 ml Dichlormethan gelöst und für 10 Min. gerührt. Ein zehnfacher Überschuss an (II) (Molenverhältnis Ligand/Rhodium = 10:1) wurde zur Rhodium-Precursorlösung gegeben und 10 Min. gerührt. Anschließend wurde die benötigte Menge an kalziniertem Siliziumdioxid Kieselgel 100 (Massenverhältnis Rhodium/Trägermaterial = 0,2 %) zugesetzt. Die erhaltene Suspension wurde für 60 Min. gerührt. Dichlormethan wurde anschließend unter Vakuum an einem Rotationverdampfer bei 700 hPa und 40°C entfernt, und das resultierende Pulver am Feinvakuum (40 Pa) über 24 h getrocknet. Bevor die katalytisch aktive Zusammensetzung zum Einsatz kam, wurde diese unter ArgonAtmosphäre gelagert.

### Erfindungsgemäßes Beispiel:

In Figur 2 ist ein Katalyseexperiment zur Hydroformylierung von C4-Gemischen (Zusammensetzung siehe Tabelle 1, Spalte 2) gezeigt. In Figur 2 ist mit - der Umsatz der linearen Butene und mit □ die n/iso-Selektivität über die Versuchslaufzeit gezeigt. Für das Katalyseexperiment wurden 3 g der katalytisch aktiven Zusammensetzung Rh-(II) eingesetzt. Der Versuch wurde bei einer Reaktionstemperatur von 393 K und einem Druck von 1 MPa; (p(1- und 2-Butene)=0,13 MPa; p(Butane)=0,05 MPa; p(H₂)=p(CO)=0,41 MPa, molares H₂/1-Buten-Verhältnis 8 : 1, Verweilzeit 43 s) durchgeführt. Zur besseren Übersichtlichkeit wurden von den ermittelten Messwerten nur jeder 10te gezeigt.

### Erfindungsgemäßes Beispiel im Vergleich zu 2. Vergleichsbeispiel:

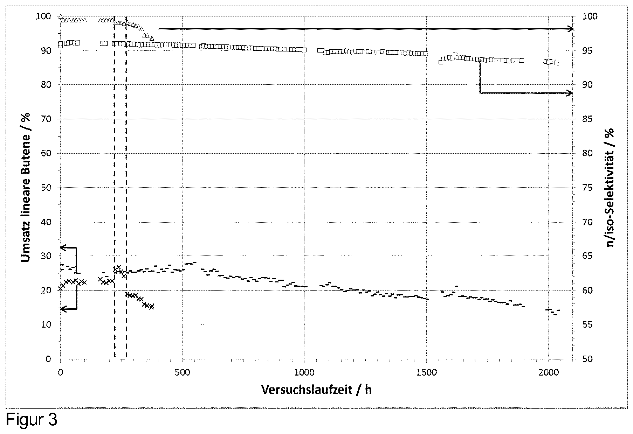

In Figur 3 sind vergleichend zwei Katalyseexperimente zur Hydroformylierung von C4-Gemischen (Zusammensetzung siehe Tabelle 1) gezeigt. In Figur 3 ist mit - der Umsatz der linearen Butene und mit □ die n/iso-Selektivität für das erfindungsgemäße Beispiel und mit x der Umsatz lineare Butene und mit Δ die n/iso-Selktivität für das 2. Vergleichsbeispiel über der Versuchslaufzeit gezeigt. Für das 2. Vergleichsbeispiel wurden 3 g der katalytisch aktiven Zusammensetzung Rh-(VIII) eingesetzt. Die Herstellung der katalytisch aktiven Zusammensetzung und Durchführung des Katalyseexperiments im 2. Vergleichsbeispiel entspricht der für das erfindungsgemäße Beispiel. Zur Herstellung der katalytisch aktiven Zusammensetzung im 2. Vergleichsbeispiel wurde Ligand (VIII) eingesetzt.

Die Reaktionstemperatur des 2. Vergleichsbeispiels betrug 373 K, zwischen 215 h und 257 h Laufzeit 393 K. Die Zusammensetzung des technischen C4-Gemischs für das 2. Vergleichsbeispiel ist in Tabelle 1 (Spalte 1) aufgeführt.

### Ergebnisse

Die erfindungsgemäße katalytisch aktive Zusammensetzung Rh-(II), mit dem unsymmetrisch substituierten Bisphosphit-Liganden (II), zeigt n/iso-Selektivität von > 90%). Insgesamt zeigt Rh-(II) eine deutlich längere Standzeit als die im Stand der Technik bekannten Zusammensetzungen. Die verwendete Masse der katalytisch aktiven Zusammensetzung bzgl. dem 1. Vergleichsbeispiel kann im erfindungsgemäßen Beispiel auf ¼ der Masse reduziert werden. Der Reaktionsverlauf mit der katalytisch aktiven Zusammensetzung Rh-(II) zeigt einen nahezu konstanten Umsatz. Vorteilhafterweise zeigt die erfindungsgemäße Zusammensetzung Rh-(II) kein Einfahrverhalten im Gegensatz zum 1. Vergleichsbeispiel mit gleichem Liganden (II), d. h. von Beginn an liegen optimale Umsätze und n/i-Selektivitäten vor. Dies ist für die großtechnische Anwendung von entscheidender Bedeutung.

Dieses Ergebnis ist überraschend, da unsymmetrische substituierte Bisphosphite gegenüber symmetrisch substituierten deutlich an Selektivität einbüßen, wie bereits in Rhodium-catalyzed Hydroformylation, ed. by P.W.N.M. van Leeuwen et C. Claver, Kluwer Academic Publishers 2006, AA Dordrecht, NL, Seite 45-46 ausgeführt wird. Des Weiteren zeichnet sich die erfindungsgemäße Zusammensetzung Rh-(II) durch eine gegenüber dem Stand der Technik bessere Temperaturstabilität aus. Die geringere verwendete Masse an katalytisch aktiver Zusammensetzung in dem erfindungsgemäßen Beispiel sowie dem 2. Vergleichsbeispiel verglichen mit der Masse an katalytisch aktiver Zusammensetzung in dem 1. Vergleichsbeispiel führen zu geringeren Umsätzen an linearen Butenen, wie ein Vergleich der Figuren 1 mit Figur 2 und Figur 3 zeigt. Das Katalyseexperiment des 2. Vergleichsbeispiels wurde bis zu einer Reaktionszeit von 215 h bei einer Reaktionstemperatur von 373 K durchgeführt. Hier zeigt sich bei gleicher Katalysatormenge im Vergleich zum erfindungsgemäßen Beispiel ein verringerter Umsatz der linearen Butene um ca. 3% bei einer Versuchszeit von 200 h (siehe Figur 3, Umsatz lineare Butene erfindungsgemäßes Beispiel (-) und 2. Vergleichsbeispiel (x)). Bei gleicher Reaktionstemperatur zeigen beide Katalyseexperimente einen identischen Umsatz an linearen Butenen von ca. 25 % (393 K, Versuchslaufzeit 215-257 h). Bei Reduktion der Reaktionstemperatur auf 373 K für das 2. Vergleichsbeispiel zeigt sich sowohl im Umsatz der linearen Butene als auch der n/iso-Selektivität ein deutliche Desaktivierung aufgrund geringerer Temperaturstabilität gegenüber der erfindungsgemäßen katalytisch aktiven Zusammensetzung. Somit zeichnet sich die erfindungsgemäße Zusammensetzung durch eine deutlich verbesserte Langzeitstabilität aus.

Zudem deutet die Langzeitstabilität von Ligand (II) auf eine bessere Stabilität von (II) gegenüber Wasser hin. Durch die höhere Reaktionstemperatur der katalytisch aktiven Zusammensetzung Rh-(II) sollte die ebenfalls thermisch induzierte Aldolkondensation verstärkt sein.

Der Ligand (II) in der katalytisch aktiven Zusammensetzung zeichnet sich durch eine deutlich bessere Langzeitstabilität als die bisher im Stand der Technik beschriebenen Liganden aus und erfüllt somit die gestellte Aufgabe. Eine optimale Langzeitstabilität der katalytisch aktiven Zusammensetzung ist insbesondere in der großtechnischen Anwendung von Bedeutung, da durch die Heterogenisierung des Katalyseprozesses im Gegensatz zur in homogener Phase durchgeführten Hydroformylierung keine Nachdosierung dieser Zusammensetzung und ihrer Komponenten möglich sind.

## Patentansprüche

1. Zusammensetzung, umfassend:
a) mindestens ein Trägermaterial, welches vorzugsweise porös ist;
b) mindestens eine Ionische Flüssigkeit;
c) mindestens ein Metall, ausgewählt aus der 9. Gruppe des Periodensystems der Elemente;
d) mindestens eine Verbindung der Formel (II): 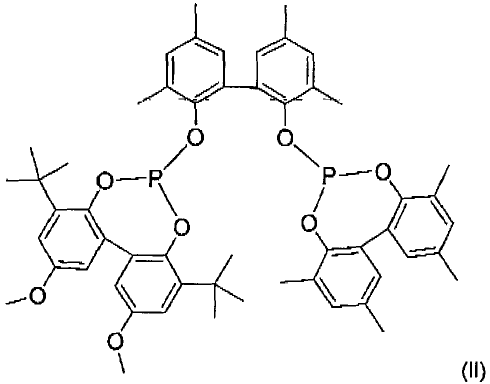
e) optional ein oder mehrere organische Amine.

2. Zusammensetzung nach Anspruch 1, wobei das poröse Trägermaterial einen oder mehrere der nachfolgenden Oberflächenparameter:
i) mittlerer Porendurchmesser in einem Bereich von 1 bis 423 nm;
ii) Porenvolumen in einem Bereich von 0,1 bis 2 ml/g;
iii) BET-Oberfläche in einem Bereich von 10 bis 2050 m²/g aufweist.

3. Zusammensetzung nach mindestens einem der Ansprüche 1 - 2, wobei das poröse Trägermaterial Oxide des Siliziums, Aluminiums, Titans, Zirkons oder Aktivkohle oder Mischungen davon aufweist oder daraus besteht.

4. Zusammensetzung nach Anspruch 3, wobei das poröse Trägermaterial Siliziumdioxid aufweist.

5. Zusammensetzung nach den Ansprüchen 1 - 4, **dadurch gekennzeichnet, dass** das Metall Rhodium ist.

6. Zusammensetzung nach den Ansprüchen 1 - 5, **dadurch gekennzeichnet, dass** als Ionische Flüssigkeit solche verwendet werden, bei denen das Anion ausgewählt ist aus der Gruppe umfassend:
Tetrafluoroborat ([BF₄]⁻), Hexafluorophosphat ([PF₆]⁻), Dicyanamid ([N(CN)₂]⁻), Bis(trifluoromethylsulfonyl)imid ([NTf₂]⁻), Tricyanomethid ([C(CN)₃]⁻), Tetracyanoborat ([B(CN)₄]⁻), Halogenide (Cl⁻, Br⁻, F⁻, I⁻), Hexafluoroantimonat ([SbF₆]⁻), Hexafluoroarsenat ([AsF₆]⁻), Sulfat ([SO₄]²⁻), Tosylat ([C₇H₇SO₃]⁻), Triflat (CF₃SO₃⁻), Nonaflat ([C₄F₉SO₃⁻), Tris-(Pentafluoroethyl)-trifluorophosphat ([PF₃(C₂F₆)₃]⁻), Thiocyanat ([SCN]⁻), Carbonat ([CO₃]²⁻), [R^{A}-COO]⁻, [R^{A}-SO₃]⁻, [R^{A}-SO₄]⁻, [R^{A}PO₄R^{B}]⁻ oder [(R^{A}-SO₂)2N]⁻ mit R^{A} und R^{B} gleich oder ungleich, jeweils ein linearer oder verzweigter 1 bis 12 kohlenstoffatome enthaltender aliphatischer oder alicyclischer Alkyl- oder Perfluoralkyl- oder ein C₅-C₁₈-substituierter Aryl-, C₅-C₁₈- substituierter Aryl-C₁-C₆-alkyl- oder C₁-C₆-Alkyl-C₅-C₁₆-substituierter Aryl-Rest ist, der durch Halogenatome substituiert sein kann;
und das Kation ausgewählt ist aus der Gruppe umfassend:
quartemäre Ammonium-Kationen der allgemeinen Formel [NR¹R²R³R⁴]⁺ mit R₁, R₂, R₃, R₄ ausgewählt aus C₁-C₈-Alkylgruppen;
Phosphonium-Kationen der allgemeinen Formel [PR¹R²R³R⁴]⁺ mit R₁, R₂, R₃, R₄ ausgewählt aus C₁-C₈-Alkylgruppen;
Imidazolium-Kationen der allgemeinen Formel (IV) 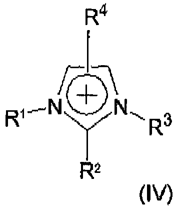 wobei der Imidazol-Kern substituiert sein kann mit wenigstens einer Gruppe R, die ausgewählt ist aus C₁-C₈-Alkyl-, C₁-C₈-Alkoxy-, C₁-C₆- substituierte Aminoalkyl-, C₅-C₁₂-substituierte Aryl- oder C₅-C₁₂- substituierte Aryl-C₁-C₆-Alkylgruppen;
Pyridinium-Kationen der allgemeinen Formel (V) 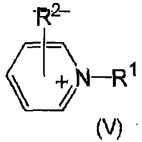 wobei der Pyridin-Kern substituiert sein kann mit wenigstens einer Gruppe R, die ausgewählt ist aus C₁-C₆-Alkyl-, C₁-C₆-Alkoxy-, C₁-C₆- substituierte Aminoalkyl-, C₆-C₁₂-substituierte Aryl- oder C₅-C₁₂- substituierte Aryl-C₁-C₆-Alkylgruppen;
Pyrazolium-Kationen der allgemeinen Formel (VI) 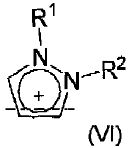 wobei der Pyrazol-Kern substituiert sein kann mit wenigstens einer Gruppe R, die ausgewählt ist aus C₁-C₆-Alkyl-, C₁-C₆-Alkoxy-, C₁-C₆- substituierte Aminoalkyl-, C₅-C₁₂- substituierte Aryl- oder C₅-C₁₂- substituierte Aryl-C₁-C₆-Alkylgruppen;
Triazolium-Kationen der allgemeinen Formel (VII) 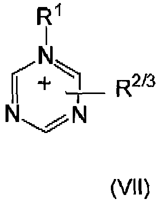 wobei der Triazol-Kern substituiert sein kann mit wenigstens einer Gruppe R, die ausgewählt ist aus
C₁-C₆-Alkyl-, C₁-C₆-Alkoxy-, C₁-C₆- substituierte Aminoalkyl-, C₅-C₁₂- substituierte Aryl- oder C₅-C₁₂- substituierte Aryl-C₁-C₆-Alkylgruppen.

7. Zusammensetzung nach den Ansprüchen 1 - 6, **dadurch gekennzeichnet, dass** das Kation der Ionischen Flüssigkeit eine Imidazolium-Struktur der allgemeinen Formel (IV) aufweist 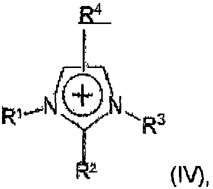 wobei der Imidazol-Kern mit wenigstens einer Gruppe R substituiert ist, ausgewählt unter C₁ - C₈-Alkylgruppen.

8. Zusammensetzung nach den Anspruch 7, **dadurch gekennzeichnet, dass** die Ionische Flüssigkeit ausgewählt ist aus der Gruppe umfassend :
a) 1-Ethyl-3-methylimidazolium bis(trifluoromethylsulfonyl)imid,
b) 1-Butyl-3-methylimidazolium hexafluorophosphat,
c) 1-Butyl-3-methylimidazolium tetrafluoroborat
d) 1-Butyl-3-methylimidazolium bis(trifluoromethylsultonyl)imid,
e) 1-Ethyl-3-methylimidazolium ethylsulfat,
f) 1-Butyl-3-methylimidazolium octylsulfat.

9. Zusammensetzung nach Anspruch 6, **dadurch gekennzeichnet, dass** die Ionische Flüssigkeit Trioctyl-Methylammonium bis(trifluoromethylsulfonyl)imid aufweist.

10. Zusammensetzung nach Anspruch 1, wobei das organische Amin mindestens einen Rest mit einer 2,2,6,6-Tetramethylpiperidineinheit gemäß Formel (III) aufweist:

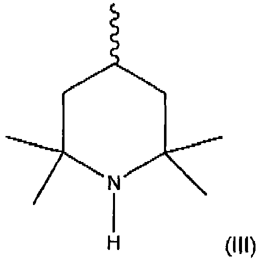

11. Zusammensetzung nach mindestens einem der Ansprüche 1 - 10, wobei das organische Amin ausgewählt ist aus Verbindungen der Formeln (IIIa) - (IIIh): 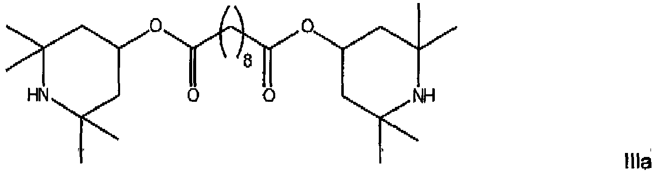 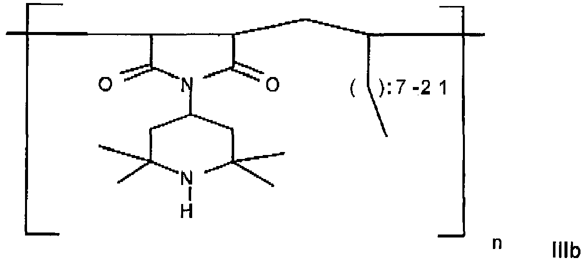 mit n = 1 bis 20 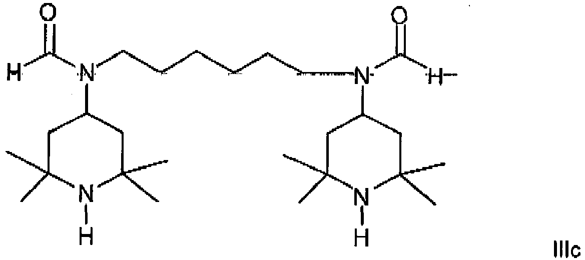 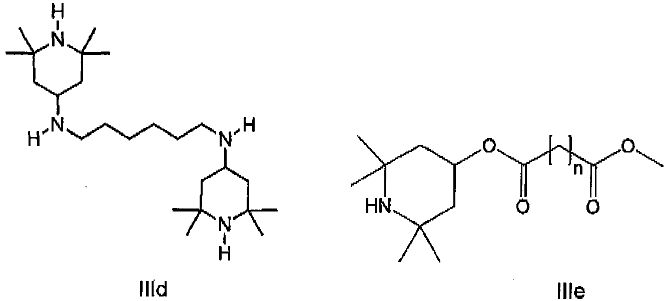 mit n = 1 bis 12 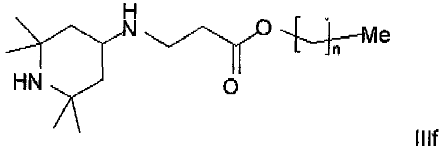 mit n = 1 bis 17 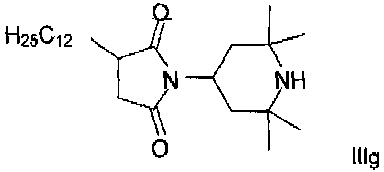 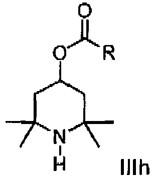 mit R = C₆- bis C₂₀-Alkyl

12. Verfahren zur Herstellung eine Zusammensetzung nach den Ansprüchen 1 bis 11, welches die Schritte aufweist:
A) vorlegen einer Vorstufe mindestens einer Verbindung eines Metalls aus der 9. Gruppe des Periodensystems der Elemente;
B) in-Kontakt-bringen mindestens einer Verbindung eines Metalls aus der 9. Gruppe des Periodensystems der Elemente mit einem molaren Überschuß bezogen auf das Metall an:
mindestens einer Verbindung
der Formel (II): 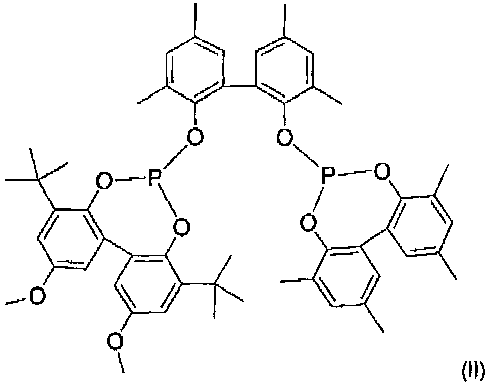 mindestens einer Ionischen Flüssigkeit nach den Ansprüchen 6 - 9;
mindestens eines organischen Amins nach den Ansprüchen 10 - 11;
unter Verwendung eines inerten Lösungsmittels
C) Zugabe mindestens eines porösen, inerten Trägermaterials nach den Ansprüchen 2 - 4 zu der unter B) generierten Mischung;
D) entfernen des inerten Lösungsmittels unter Erhalt der Zusammensetzung.

13. Verwendung einer Zusammensetzung nach den Ansprüchen 1 - 11 als Katalysator in einem Verfahren zur Hydroformylierung von ungesättigten Verbindungen.

14. Verfahren zu Hydroformylierung von ungesättigten Verbindungen, **dadurch gekennzeichnet, dass** eine Zusammensetzung nach den Ansprüchen 1 - 11, als Katalysator eingesetzt wird.

15. Verfahren nach Anspruch 14, **dadurch gekennzeichnet, dass** die ungesättigten Verbindungen ausgewählt sind aus C₂ - C₄ - Olefinen und deren technischen Gemischen:

16. Verfahren nach Anspruch 15, **dadurch gekennzeichnet, dass** die ungesättigten Verbindungen ausgewählt sind aus Ethen, Propen, Butenen oder deren technischen Gemischen.

17. Verfahren nach Anspruch 16, **dadurch gekennzeichnet, dass** die Hydroformylierung:
a) in mindestens einem Festbettreaktor;
b) in einem Temperaturbereich von 353,15 K bis 433,15 K, bevorzugt von 363,15 K bis 413,15 K;
c) in einem Druckbereich von 0,5 MPa bis 1,1 MPa;
d) mit einem molaren Verhältnis von H₂ zu 1-Buten zwischen 1:1 bis 15:1, bevorzugt zwischen 6:1 bis 8:1 durchgeführt wird,
wobei die Abtrennung der Reaktionsprodukte über die Gasphase unter Verwendung eines Gasgemisches umfassend CO und H₂ erfolgt.

## Claims

1. Composition comprising:
a) at least one support material which is preferably porous;
b) at least one ionic liquid;
c) at least one metal selected from the 9^{th} group of the Periodic Table of the Elements;
d) at least one compound of the formula (II): 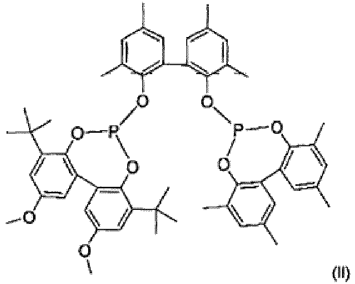
e) optionally one or more organic amines.

2. Composition according to Claim 1, wherein the porous support material has one or more of the following surface parameters:
i) average pore diameter in the range from 1 to 423 nm;
ii) pore volume in the range from 0.1 to 2 ml/g;
iii) BET surface area in the range from 10 to 2050 m²/g.

3. Composition according to at least one of Claims 1-2, wherein the porous support material comprises or consists of oxides of silicon, of aluminium, of titanium, of zirconium or activated carbon or a mixture thereof.

4. Composition according to Claim 3, wherein the porous support material comprises silicon dioxide.

5. Composition according to Claims 1-4, **characterized in that** the metal is rhodium.

6. Composition according to Claims 1-5, **characterized in that** an ionic liquid in which the anion is selected from the group consisting of:
tetrafluoroborate ([BF₄]⁻), hexafluorophosphate ([PF₆]⁻), dicyanamide ([N(CN)₂]⁻), bis(trifluoromethylsulphonyl) imide ([NTf₂]⁻), tricyanomethide ([C(CN)₃]⁻), tetracyanoborate ([B(CN)₄]⁻), halides (Cl⁻, Br⁻, F⁻, I⁻), hexafluoroantimonate ([SbF₆]⁻), hexafluoroarsenate ([AsF₆]⁻), sulphate ([SO₄]²⁻), tosylate ([C₇H₇SO₃]⁻), triflate (CF₃SO₃⁻), nonaflate ([C₄F₉SO₃⁻), tris (pentafluoroethyl)trifluorophosphate ([PF₃(C₂F₆)₃]⁻), thiocyanate ([SCN]⁻), carbonate ([CO₃]²⁻), [R^{A}-COO]⁻, [R^{A}-SO₃]⁻, [R^{A}-SO₄]⁻, [R^{A}PO₄R^{B}]⁻ or [(R^{A}-SO₂)2N]⁻ where R^{A} and R^{B} are identical or different and are each a linear or branched aliphatic or alicyclic alkyl or perfluoroalkyl radical containing from 1 to 12 carbon atoms or a C₅-C₁₈-substituted aryl, C₅-C₁₈-substituted aryl, C₁-C₆-alkyl or C₁-C₆-alkyl-C₈-C₁₈-substituted aryl radical which may be substituted by halogen atoms; and the cation is selected from the group consisting of:
quaternary ammonium cations of the general formula [NR¹R²R³R⁴]⁺ where R₁, R₂, R₃, R₄ are selected from among C₁-C₈-alkyl groups;
phosphonium cations of the general formula [PR¹R²R³R⁴]⁺ where R₁, R₂, R₃, R₄ are selected from among C₁-C₈-alkyl groups;
imidazolium cations of the general formula (IV) 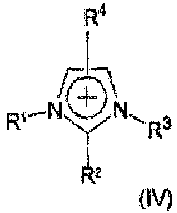 where the imidazole ring can be substituted by at least one group R selected from among C₁-C₈-alkyl, C₁-C₈-alkoxy, C₁-C₆-substituted aminoalkyl, C₈-C₁₂-substituted aryl or C₅-C₁₂-substituted-aryl-C₁-C₆-alkyl groups;
pyridinium cations of the general formula (V) 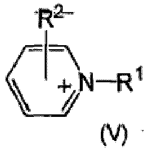 where the pyridine ring can be substituted by at least one group R selected from among C₁-C₆-alkyl, C₁-C₆-alkoxy, C₁-C₆-substituted aminoalkyl, C₅-C₁₂-substituted aryl or C₅-C₁₂-substituted aryl-C₁-C₆-alkyl groups;
pyrazolium cations of the general formula (VI) 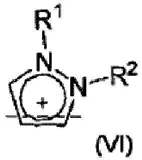 where the pyrazole ring can be substituted by at least one group R selected from among C₁-C₆-alkyl, C₁-C₆-alkoxy, C₁-C₆-substituted aminoalkyl, C₅-C₁₂-substituted aryl or C₅-C₁₂-substituted aryl-C₁-C₆-alkyl groups;
triazolium cations of the general formula (VII) 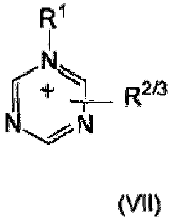 where the triazole ring can be substituted by at least one group R selected from among C₁-C₆-alkyl, C₁-C₆-alkoxy, C₁-C₆-substituted aminoalkyl, C₅-C₁₂-substituted aryl or C₅-C₁₂-substituted aryl-C₁-C₆-alkyl groups,
is used as ionic liquid.

7. Composition according to Claims 1-6, **characterized in that** the cation of the ionic liquid has an imidazolium structure of the general formula (IV) 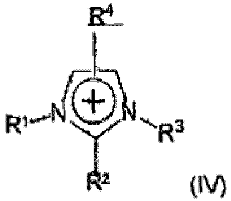 where the imidazole ring is substituted by at least one group R selected from among C₁-C₈-alkyl groups.

8. Composition according to Claim 7, **characterized in that** the ionic liquid is selected from the group consisting of:
a) 1-ethyl-3-methylimidazolium bis(trifluoromethylsulphonyl)imide,
b) 1-butyl-3-methylimidazolium hexafluorophosphate,
c) 1-butyl-3-methylimidazolium tetrafluoroborate,
d) 1-butyl-3-methylimidazolium bis(trifluoromethylsulphonyl)imide,
e) 1-ethyl-3-methylimidazolium ethylsulphate,
f) 1-butyl-3-methylimidazolium octylsulphate.

9. Composition according to Claim 6, **characterized in that** the ionic liquid comprises trioctylmethylammonium bis(trifluoromethylsulphonyl)imide.

10. Composition according to Claim 1, wherein the organic amine has at least one radical having a 2,2,6,6-tetramethylpiperidine unit of the formula (III) :

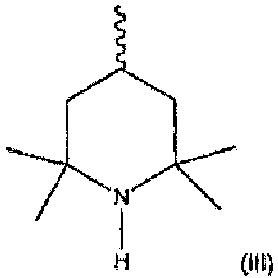

11. Composition according to at least one of Claims 1-10, wherein the organic amine is selected from among compounds of the formulae (IIIa) - (IIIh) : 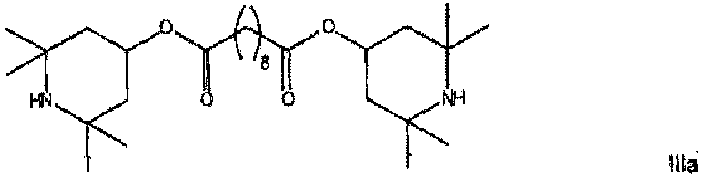 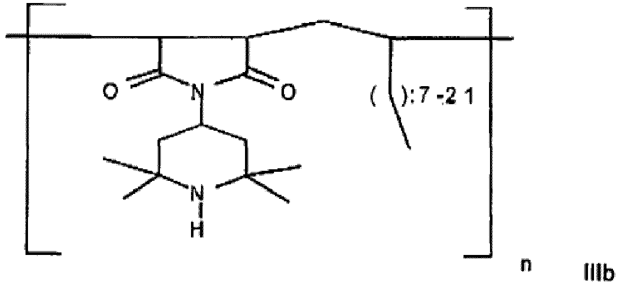 where n = 1 to 20 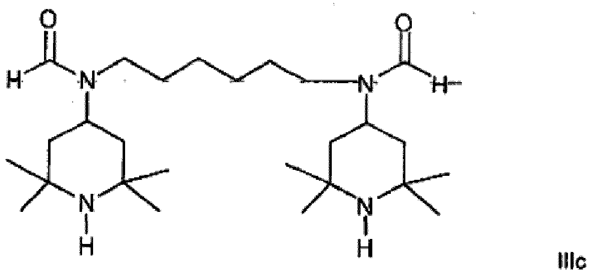 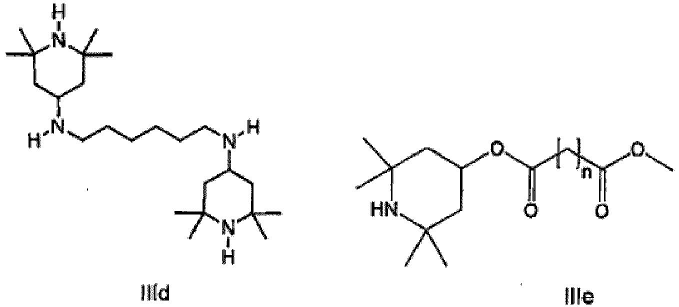 where n = 1 to 12 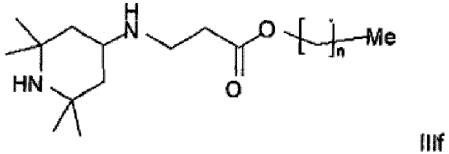 where n = 1 to 17 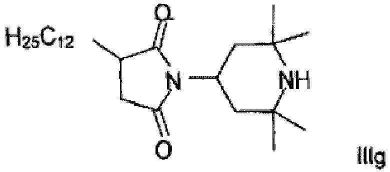 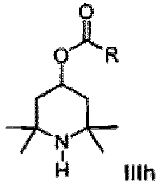 where R = C₆-C₂₀-alkyl.

12. Process for producing a composition according to Claims 1 to 11, which comprises the steps:
A) provision of a precursor of at least one compound of a metal of the 9^{th} group of the Periodic Table of the Elements;
B) contacting of at least one compound of a metal of the 9^{th} group of the Periodic Table of the Elements with a molar excess based on the metal of:
at least one compound of the formula (II): 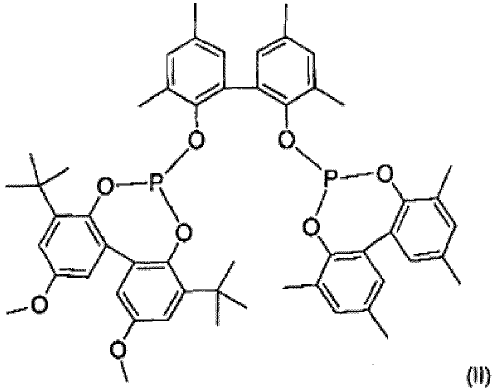 at least one ionic liquid according to Claims 6-9;
at least one organic amine according to Claims 10-11; using an inert solvent;
C) addition of at least one porous, inert support material according to Claims 2-4 to the mixture generated under B);
D) removal of the inert solvent to give the composition.

13. Use of a composition according to Claims 1-11 as catalyst in a process for the hydroformylation of unsaturated compounds.

14. Process for the hydroformylation of unsaturated compounds, **characterized in that** a composition according to Claims 1-11 is used as catalyst.

15. Process according to Claim 14, **characterized in that** the unsaturated compounds are selected from among C₂-C₄-olefins and industrial mixtures thereof.

16. Process according to Claim 15, **characterized in that** the unsaturated compounds are selected from among ethene, propene, butenes and industrial mixtures thereof.

17. Process according to Claim 16, **characterized in that** the hydroformylation is carried out:
a) in at least one fixed-bed reactor;
b) in a temperature range from 353.15 K to 433.15 K, preferably from 363.15 K to 413.15 K;
c) in a pressure range from 0.5 MPa to 1.1 MPa;
d) at a molar ratio of H₂ to 1-butene in the range from 1:1 to 15:1, preferably from 6:1 to 8:1,
where the reaction products are separated off via the gas phase using a gas mixture comprising CO and H₂.

## Revendications

1. Composition, comprenant :
a) au moins un matériau support, qui est de préférence poreux ;
b) au moins un liquide ionique ;
c) au moins un métal, choisi dans le groupe 9 du tableau périodique des éléments ;
d) au moins un composé de formule (II) : 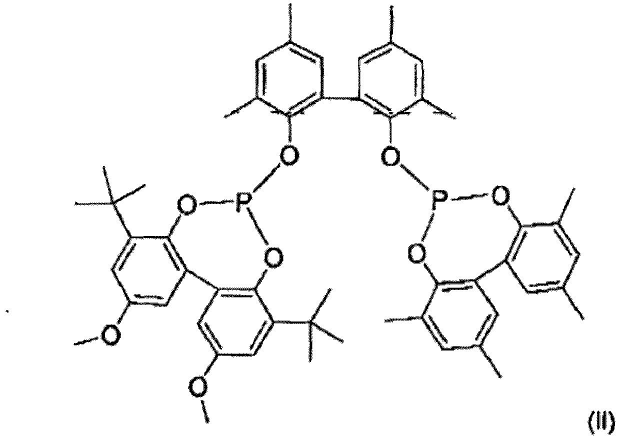
e) éventuellement une ou plusieurs amines organiques.

2. Composition selon la revendication 1, dans laquelle le matériau support poreux présente un ou plusieurs des paramètres de surface suivants :
i) un diamètre de pore moyen dans une plage allant de 1 à 423 nm ;
ii) un volume de pore dans une plage allant de 0,1 à 2 ml/g ;
iii) une surface BET dans une plage allant de 10 à 2050 m²/g.

3. Composition selon au moins l'une quelconque des revendications 1 à 2, dans laquelle le matériau support poreux comprend des oxydes de silicium, d'aluminium, de titane, de zirconium ou du charbon actif ou leurs mélanges ou en est constitué.

4. Composition selon la revendication 3, dans laquelle le matériau support poreux comprend du dioxyde de silicium.

5. Composition selon les revendications 1 à 4, **caractérisée en ce que** le métal est le rhodium.

6. Composition selon les revendications 1 à 5, **caractérisée en ce qu'**on utilise des liquides ioniques dans lesquels l'anion est choisi dans le groupe comprenant :
le tétrafluoroborate ([BF₄]⁻), l'hexafluorophosphate ([PF₆]⁻), le dicyanamide ([N(CN)₂]⁻), le bis(trifluorométhylsulfonyl)imide ([NTf₂]⁻), le tricyanométhide ([C(CN)₃]⁻), le tétracyanoborate ([B(CN)₄]⁻), les halogénures (Cl⁻, Br⁻, F⁻, I⁻), l'hexafluoroantimonate ([SbF₆]⁻), l'hexafluoroarsenate ([AsF₆]⁻), le sulfate ([SO₄]²⁻), le tosylate ([C₇H₇SO₃]⁻), le triflate (CF₃SO₃⁻), le nonaflate ([C₄F₉SO₃]⁻), le tris-(pentafluoroéthyl)-trifluorophosphate ([PF₃(C₂F₅)₃]⁻), le thiocyanate ([SCN]⁻), le carbonate ([CO₃]²⁻), [R^{A}-COO]⁻, [R^{A}-SO₃]⁻, [R^{A}-SO₄]⁻, [R^{A}PO₄R^{B}]⁻ ou [(R^{A}-SO₂)2N]⁻ avec R^{A} et R^{B} identiques ou différents, représentant chacun un radical alkyle ou perfluoroalkyle aliphatique ou alicyclique linéaire ou ramifié contenant 1 à 12 atomes de carbone, ou un radical aryle en C₈-C₁₈ substitué, aryle en C₅-C₁₈ substitué-alkyle en C₁-C₆ ou alkyle en C₁-C₆-aryle en C₅-C₁₈ substitué, qui peut être substitué par des atomes d'halogène ;
et le cation est choisi dans le groupe comprenant :
les cations ammonium quaternaire de formule générale [NR¹R²R³R⁴]⁺ avec R₁, R₂, R₃, R₄ choisis parmi les groupes alkyle en C₁-C₈ ;
les cations phosphonium de formule générale [PR¹R²R³R⁴]⁺ avec R₁, R₂, R₃, R₄ choisis parmi les groupes alkyle en C₁-C₈ ;
les cations imidazolium de formule générale (IV) 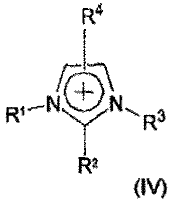 dans laquelle le noyau imidazole peut être substitué avec au moins un groupe R, qui est choisi parmi les groupes alkyle en C₁-C₈, alcoxy en C₁-C₆, aminoalkyle en C₁-C₆ substitué, aryle en C₅-C₁₂ substitué ou aryle en C₅-C₁₂ substitué-alkyle en C₁-C₆ ;
les cations pyridinium de formule générale (V) 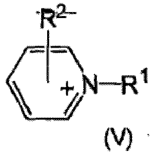 dans laquelle le noyau pyridine peut être substitué avec au moins un groupe R, qui est choisi parmi les groupes alkyle en C₁-C₆, alcoxy en C₁-C₆, aminoalkyle en C₁-C₆ substitué, aryle en C₅-C₁₂ substitué ou aryle en C₅-C₁₂ substitué-alkyle en C₁-C₆ ;
les cations pyrazolium de formule générale (VI) 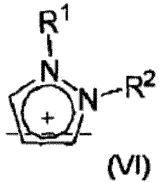 dans laquelle le noyau pyrazole peut être substitué avec au moins un groupe R, qui est choisi parmi les groupes alkyle en C₁-C₆, alcoxy en C₁-C₆, aminoalkyle en C₁-C₆ substitué, aryle en C₅-C₁₂ substitué ou aryle en C₅-C₁₂ substitué-alkyle en C₁-C₆ ;
les cations triazolium de formule générale (VII) 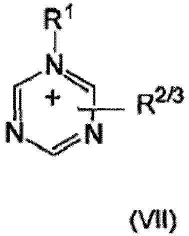 dans laquelle le noyau triazole peut être substitué avec au moins un groupe R, qui est choisi parmi les groupes alkyle en C₁-C₆, alcoxy en C₁-C₆, aminoalkyle en C₁-C₆ substitué, aryle en C₅-C₁₂ substitué ou aryle en C₅-C₁₂ substitué-alkyle en C₁-C₆.

7. Composition selon les revendications 1 à 6, **caractérisée en ce que** le cation du liquide ionique comprend une structure imidazolium de formule générale (IV) 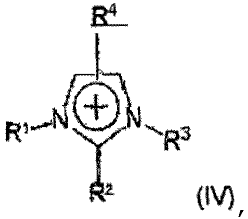 dans laquelle le noyau imidazole est substitué avec au moins un groupe R, qui est choisi parmi les groupes alkyle en C₁-C₈.

8. Composition selon la revendication 7, **caractérisée en ce que** le liquide ionique est choisi dans le groupe comprenant :
a) le bis(trifluorométhylsulfonyl)imide de 1-éthyl-3-méthylimidazolium,
b) l'hexafluorophosphate de 1-butyl-3-méthylimidazolium,
c) le tétrafluoroborate de 1-butyl-3-méthylimidazolium,
d) le bis(trifluorométhylsulfonyl)imide de 1-butyl-3-méthylimidazolium,
e) l'éthylsulfate de 1-éthyl-3-méthylimidazolium,
f) l'octylsulfate de 1-butyl-3-méthylimidazolium.

9. Composition selon la revendication 6, **caractérisée en ce que** le liquide ionique comprend du bis(trifluorométhylsulfonyl)imide de trioctyl-méthylammonium.

10. Composition selon la revendication 1, dans laquelle l'amine organique comprend au moins un radical contenant une unité 2,2,6,6-tétraméthylpipéridine selon la formule (III) :

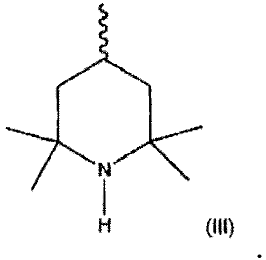

11. Composition selon au moins l'une quelconque des revendications 1 à 10, dans laquelle l'amine organique est choisie parmi les composés de formule (IIIa) à (IIIh) : 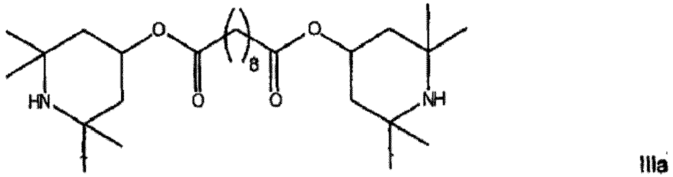 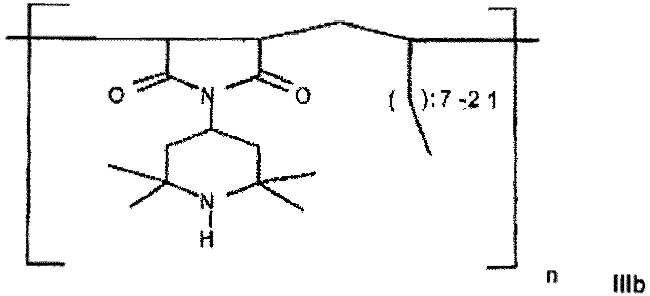 avec n = 1 à 20, 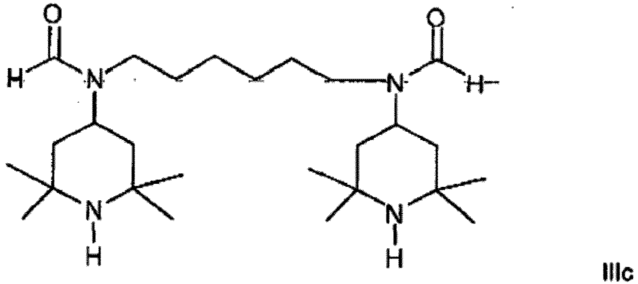 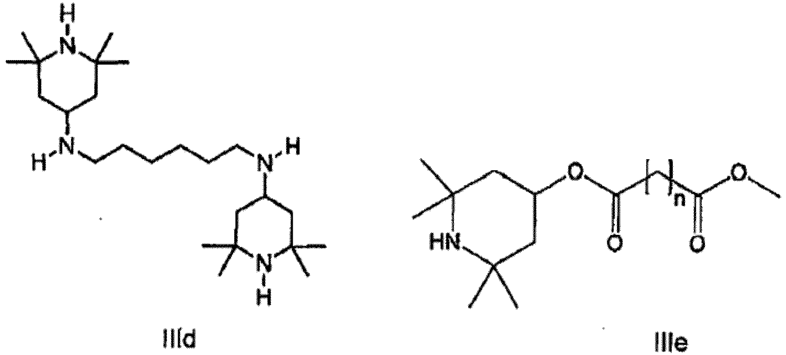 avec n = 1 à 12, 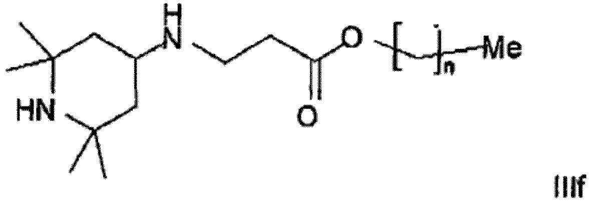 avec n = 1 à 17 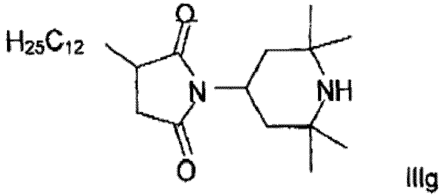 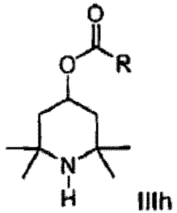 avec R = alkyle en C₆ à C₂₀.

12. Procédé de fabrication d'une composition selon les revendications 1 à 11, qui comprend les étapes suivantes :
A) le chargement d'un précurseur d'au moins un composé d'un métal du groupe 9 du tableau périodique des éléments ;
B) la mise en contact d'au moins un composé d'un métal du groupe 9 du tableau périodique des éléments avec un excès molaire par rapport au métal de :
au moins un composé de formule (II) : 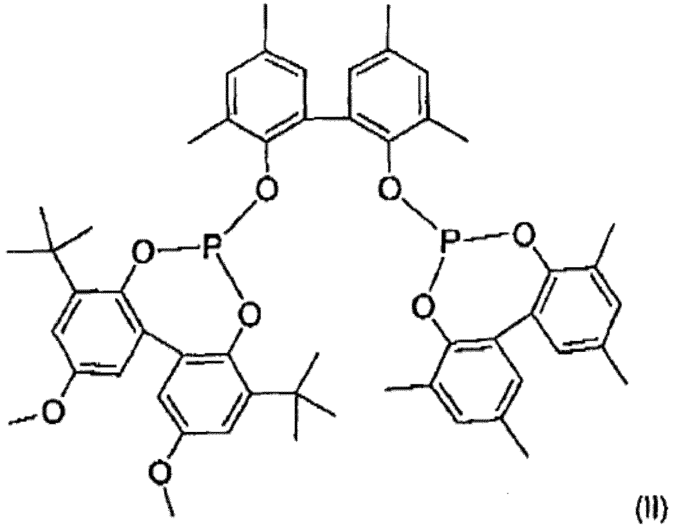 au moins un liquide ionique selon les revendications 6 à 9 ;
au moins une amine organique selon les revendications 10 à 11 ;
en utilisant un solvant inerte,
C) l'ajout d'au moins un matériau support inerte poreux selon les revendications 2 à 4 au mélange généré en B) ;
D) l'élimination du solvant inerte pour obtenir la composition.

13. Utilisation d'une composition selon les revendications 1 à 11 en tant que catalyseur dans un procédé d'hydroformylation de composés insaturés.

14. Procédé d'hydroformylation de composés insaturés, **caractérisé en ce qu'**une composition selon les revendications 1 à 11 est utilisée en tant que catalyseur.

15. Procédé selon la revendication 14, **caractérisé en ce que** les composés insaturés sont choisis parmi les oléfines en C₂-C₄ et leurs mélanges techniques.

16. Procédé selon la revendication 15, **caractérisé en ce que** les composés insaturés sont choisis parmi l'éthène, le propène, les butènes ou leurs mélanges techniques.

17. Procédé selon la revendication 16, **caractérisé en ce que** l'hydroformylation est réalisée :
a) dans au moins un réacteur à lit fixe ;
b) dans une plage de température allant de 353,15 K à 433,15 K, de préférence de 363,15 K à 413,15 K ;
c) dans une plage de pression allant de 0,5 MPa à 1,1 MPa ;
d) avec un rapport molaire H₂ sur 1-butène compris entre 1:1 et 15:1, de préférence entre 6:1 et 8:1,
la séparation des produits de réaction ayan lieu par le biais de la phase gazeuse en utilisant un mélange gazeux comprenant CO et H₂.
